# EUROPEAN PATENT SPECIFICATION

(11) **EP 0 429 538 B1**
(45) Date of publication and mention of the grant of the patent: **07.11.2001**
(21) Application number: 89910282.6
(22) Date of filing: 17.08.1989
(51) Int. Cl.: C12N 15/67, A01H 5/00

(54) **PLANT ELONGATION FACTOR, PROMOTERS, CODING SEQUENCES AND USES**
PFLANZENVERLÄNGERUNGSFAKTOR, PROMOTOREN, KODIERENDE SEQUENZEN UND VERWENDUNGEN
FACTEUR D'ELONGATION DE PLANTES, PROMOTEURS, SEQUENCES CODANTES ET UTILISATIONS

(30) Priority: 18.08.1988 US 234187; 07.03.1989 US 335133
(43) Date of publication of application: 05.06.1991
(73) Proprietor: Calgene LLC, Davis, California 95616 (US)
(72) Inventor: SHEWMAKER, Christine, K., Woodland, CA 95695 (US); HIATT, William, R., Davis, CA 95616 (US); POKALSKY, Ann, R., East Meadow, NY 11554 (US)
(74) Representative: Armitage, Ian Michael
(86) International application number: US8903536
(87) International publication number: WO9002172

(56) References cited:
- EP-A- 0 240 208
- WO-A-88/02402
- US-A- 4 656 131
- JOURNAL OF CELLULAR BIOCHEMISTRY, suppl. 12C, 1988, UCLA SYMPOSIA ON MOLECULAR & CELLULAR BIOLOGY, ABSTRACTS OF THE 17TH ANNUAL MEETINGS, 28th February - 10th April 1988, page 148, abstract no. L 043, Alan R. Liss, Inc., New York, US; W.R. HIATT et al.: "Expression of selected genes during tomato fruit maturation and ripening"
- IDEM

## Description

### INTRODUCTION

### Technical Field

The field of this invention relates to compositions and methods for modification of plant phenotype by preferential transcription of a gene of interest in specific tissue (s).

### Background

With an increasing population, there is a continual demand for an increasing supply of food staples. In addition, in the wealthy societies, there is an expanding interest in food variety and improvement in organoleptic properties. Much of the revolution in agriculture in the increase of land utilization has been a result of the use of fertilizers. However, the use of fertilizers is a mixed blessing, in that the soil becomes ultimately less productive and the ground water becomes contaminated, resulting in eutrophy in various bodies of water.

With the ability to introduce DNA into plants, there is the potential to modify the phenotype of the plant. Various modifications have already been made and continue to be made. One modification of interest has been herbicide resistance. By providing for the crop being herbicide resistant, one can provide for clear discrimination between the crop and weeds. Thus, the weeds may be destroyed, so that there is no diversion of nutrient and water to unproductive plants.

Initially, constitutive production of herbicide resistance was introduced into plants, so that there was a substantially constant amount of the herbicide resistant product being produced throughout the plant, regardless of the utility of such capability in various tissues (Comai et al. (1985) Nature 317:741-744). A further improvement resulted in the modification in the herbicide resistant gene with a leader sequence which allowed for transport of the gene into the chloroplast where the herbicide target was in the chloroplast (della-Cioppa et al. (1987) Bio/Technology 5:579-584 and WO88/02402). Since some herbicides inhibit enzymes present in the chloroplast providing for a herbicide-resistant enzyme in the chloroplast would maintain the enzyme function at the natural site where it is utilized. Other improvements have involved introduction of insect resistance, where an insecticidal protein existing naturally in bacterial organisms is introduced into plants for expression and resistance of the plant to insects (Fischhoff et al. (1987) Bio/Technology 5:807-813).

In many instances, there is a particular interest in protecting rapidly dividing cells or rapidly growing tissue against stress or other detrimental factors. For example, the site of action of various herbicides is rapidly dividing cells. Insects frequently target young tender tissue for attack resulting in injury at this site. Certain plant diseases are particularly severe with young rapidly dividing cells. Also, the tender new tissue is most sensitive to stresses such as frost, so that enhanced production of products which protect against frost or inhibition of endogenous products which enhance the sensitivity to frost is of primary interest in such tissue.

There is, therefore, substantial interest in being able to target particular products to rapidly dividing cells in young tissue. In some instances, the protein which is produced may be deleterious to other tissue at elevated levels, so that one wishes to restrict expression to the rapidly dividing cells. In other situations, the product will have no utility at other sites, so that the energy of the plant is wasted, reducing growth efficiency. Toward this end, it is important to identify genes and their promoters which will allow for expression in selected tissue(s) at a desired high level, while providing for low-or no-level of expression in other tissue.

### Relevant Literature

Genes encoding elongation factor-1α from several species have been cloned and sequenced. These include yeast (Nagata, et al., EMBO J. (1984) 3:1825-1830); the brine shrimp Artemia salina; (van Hemert, et al., FEBS Lett. (1983) 157:295-299; the fungus Mucor racemosus (Linz, et al., J. Biol. Chem. (1986) 261:15022-15029); and man (Brands, et al., Eur. J. Biochem. (1986) 155:167-171). For a review of elongation factor-1α (EF-1α) protein, see Ejiri, Methods in Enzymology (1986), 118:140-153. Journal of Cellular Biochemistry, Suppl. 12C, April 1988, Abstract 043, W.R. Hiatt et al : "Expression of selected genes during tomato fruit maturation and ripening" mentions that genomic clones for elongation factor-1α (ET-1α), 2A11, and polygalacturonase (PG) have been isolated and characterised.

### BRIEF DESCRIPTION OF THE DRAWINGS

Figure 1 shows the sequence of LeEF-1.

Figure 2 shows amino acid comparisons between tomato and other EF-1α proteins. 1 = L. esculentum (tomato; LeEF-1 this paper); 2 = A. salina (brine shrimp) (van Hemert et al., 1984); 3 = H. sapien (man) (Brands et al., 1986); 4 = M. racemosus (a dimorphic fungus) (Linz et al., 1985); 5 = S. cerevisiae (yeast) (Nagata et al., 1984). Boxes indicate residues which are homologous tomato EF-1α.

Figure 3 shows the sequence of the EF-1α genomic clone, 61-1 from tomato.

Figure 4 shows the sequence of the EF-1α genomic clone, clone A, also referred to as LeEF-A.

Figure 5 is a graph representing a comparison of expression levels of various constructs of a flurometric GUS assay in transformed tobacco plants. Two EF-1α-GUS constructs (pCGN2157 and pCGN2158) are represented and compared to a mas-GUS construct (pCGN7001) and repeated testing of a single double 35S-GUS clone (pCGN7320).

### SUMMARY OF THE INVENTION

Novel DNA transcription constructs, together with methods for their preparation and use, are provided for modifying the phenotype of plant cells and tissues. Also provided is a DNA sequence for preparation of the transcription constructs. When transformed into a plant cell, the transcription constructs provide for a transcription pattern of a gene of interest associated with that of elongation factor-1α (EF-1α). The DNA comprises the 5' non-coding regions associated with the plant EF-1α.

The entire coding region for the plant EF-1α and also the 3' non-coding region are described. DNA transcription constructs are prepared which include the transcriptional and translational regulatory region of EF-1α and a gene of interest other than the EF-1α gene under the regulatory control of the regulatory region and a termination region functional in a plant cell. The transcriptional construct can be used to insert any of a variety of endogenous or exogenous genes into a plant cell for high level production of transcription products, as well as translation products associated with varying the phenotype of specific plant cells and tissue.

### DESCRIPTION OF THE SPECIFIC EMBODIMENTS

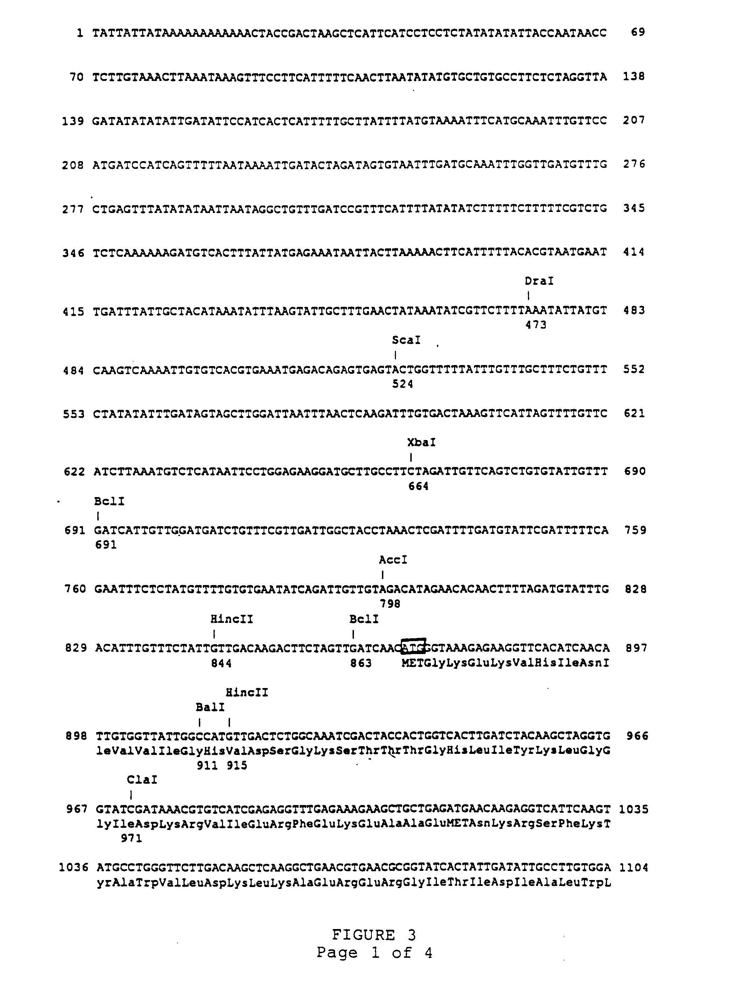

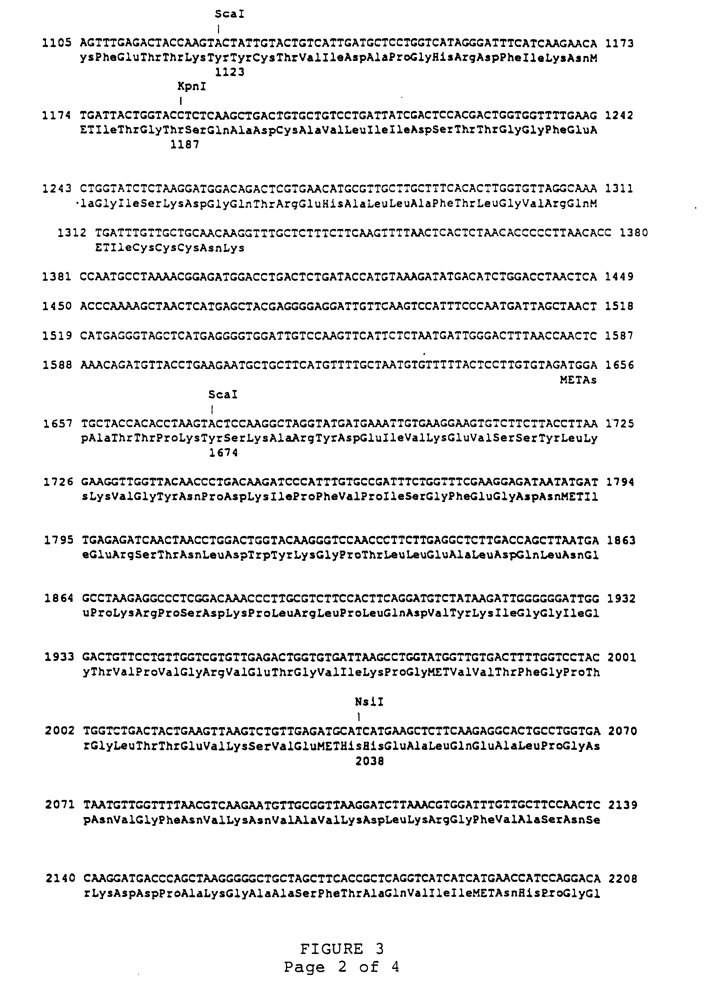

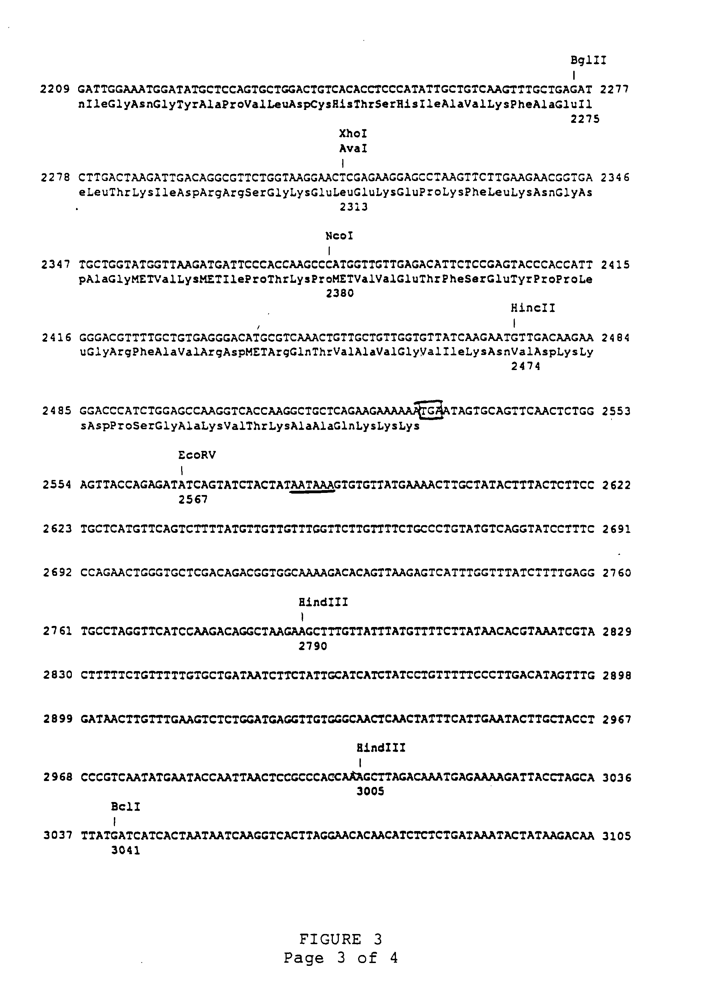

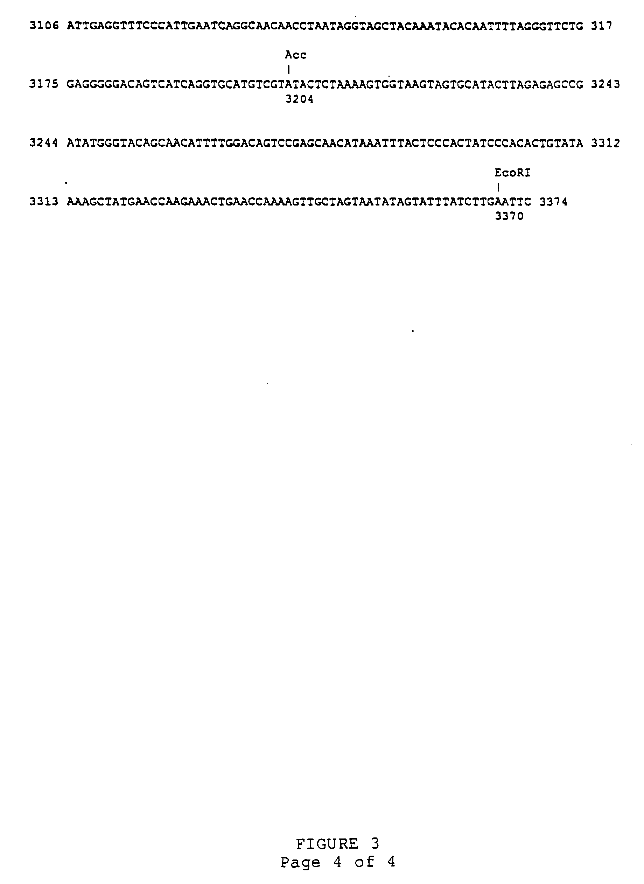

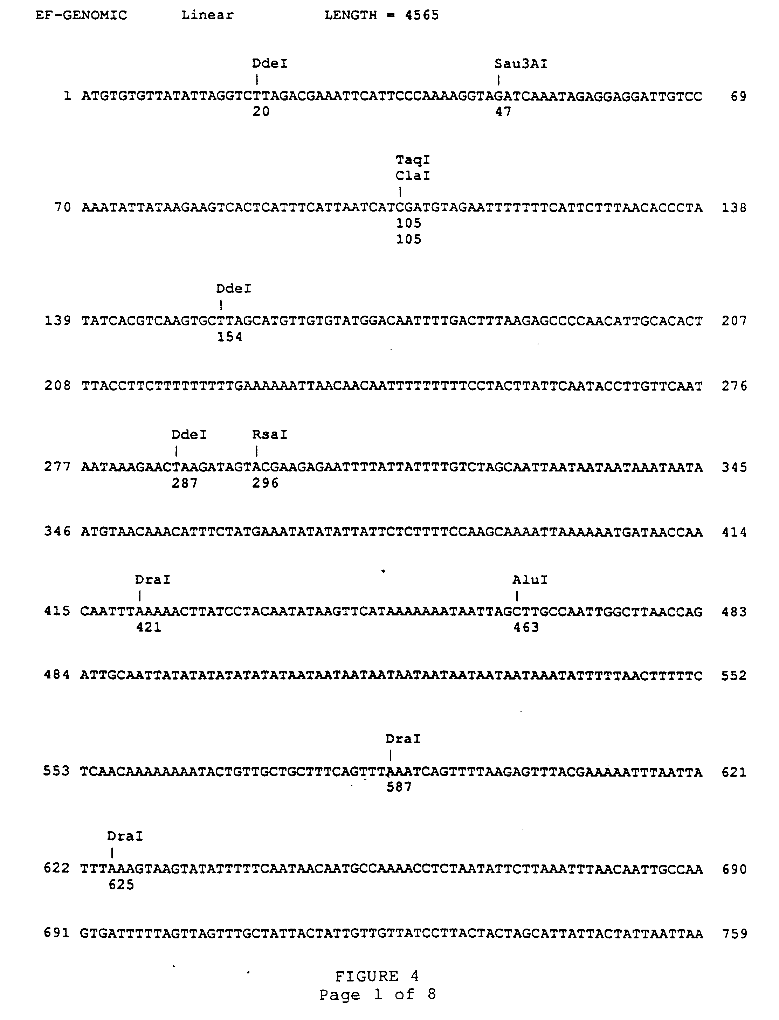

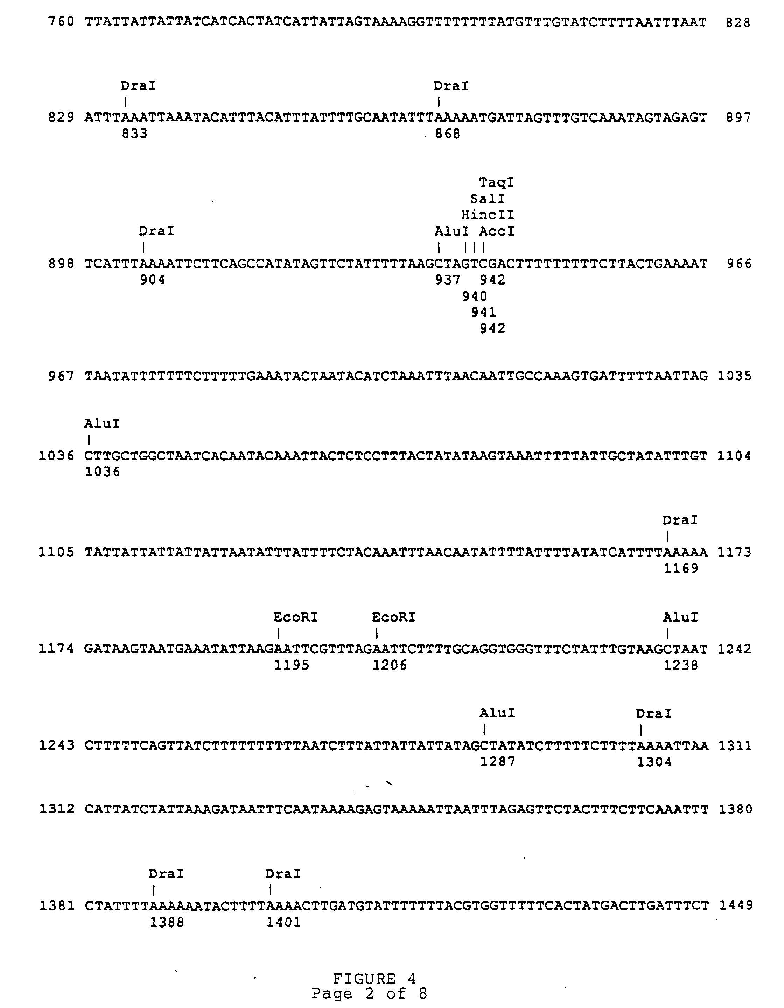

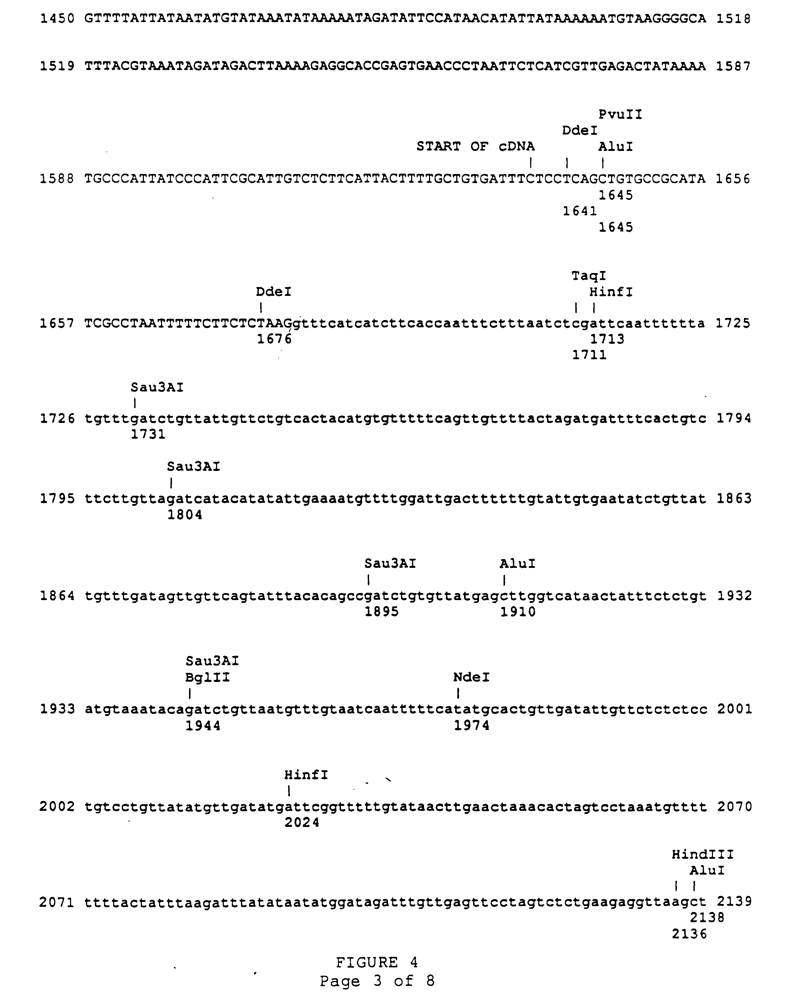

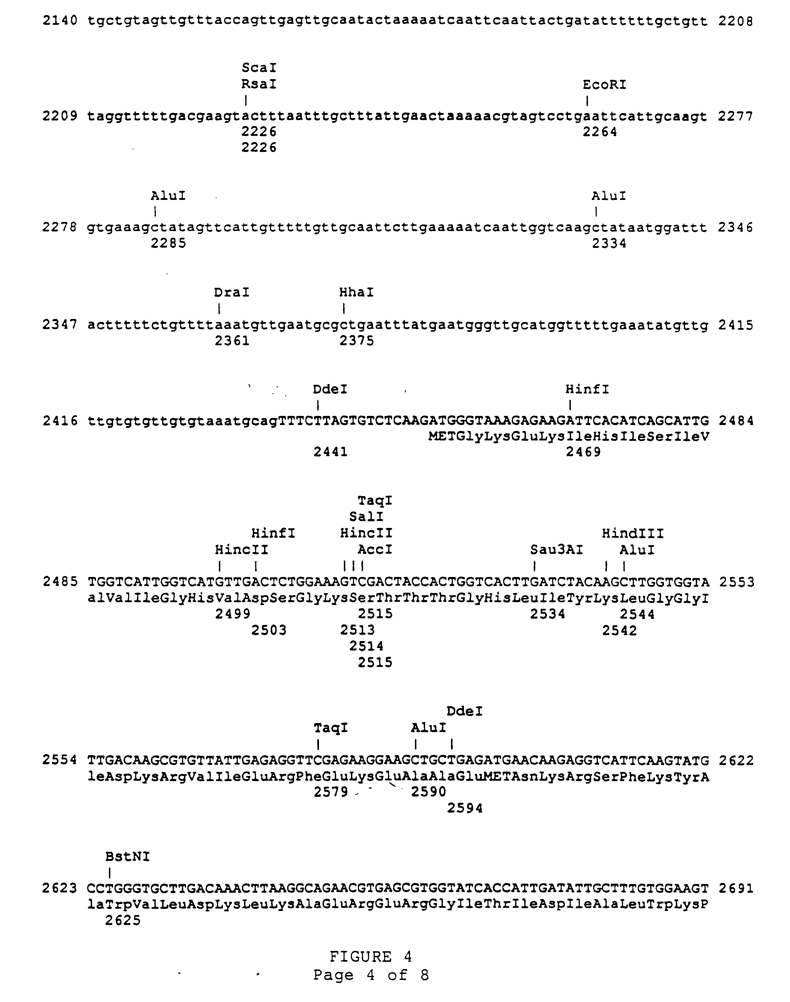

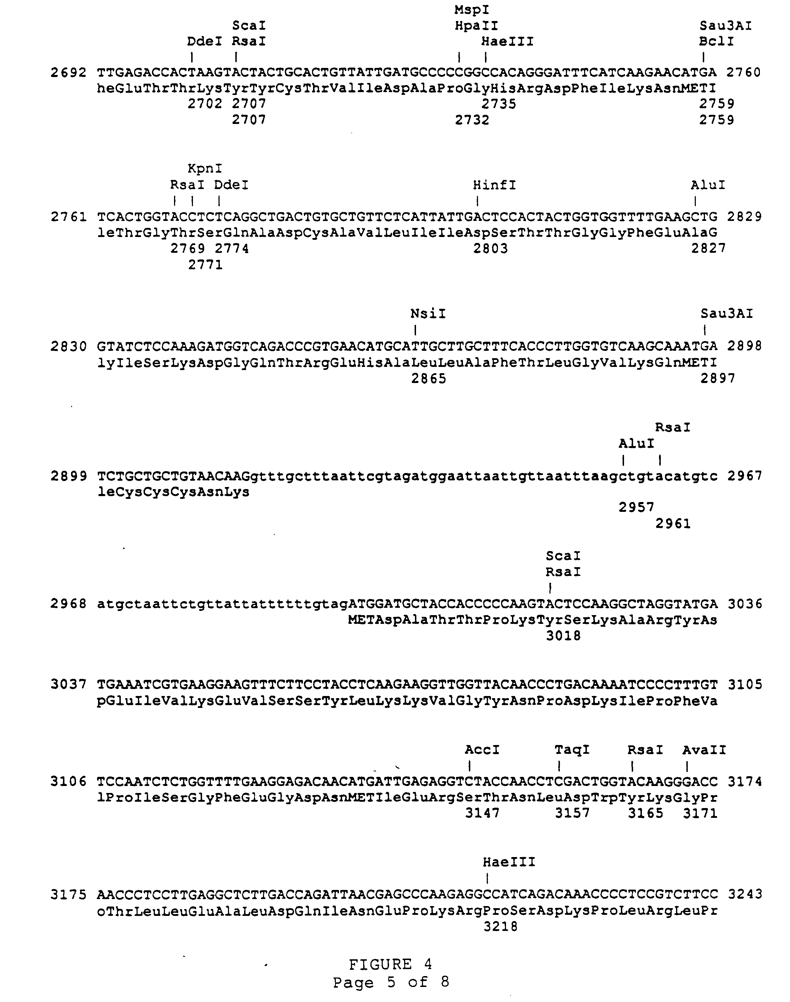

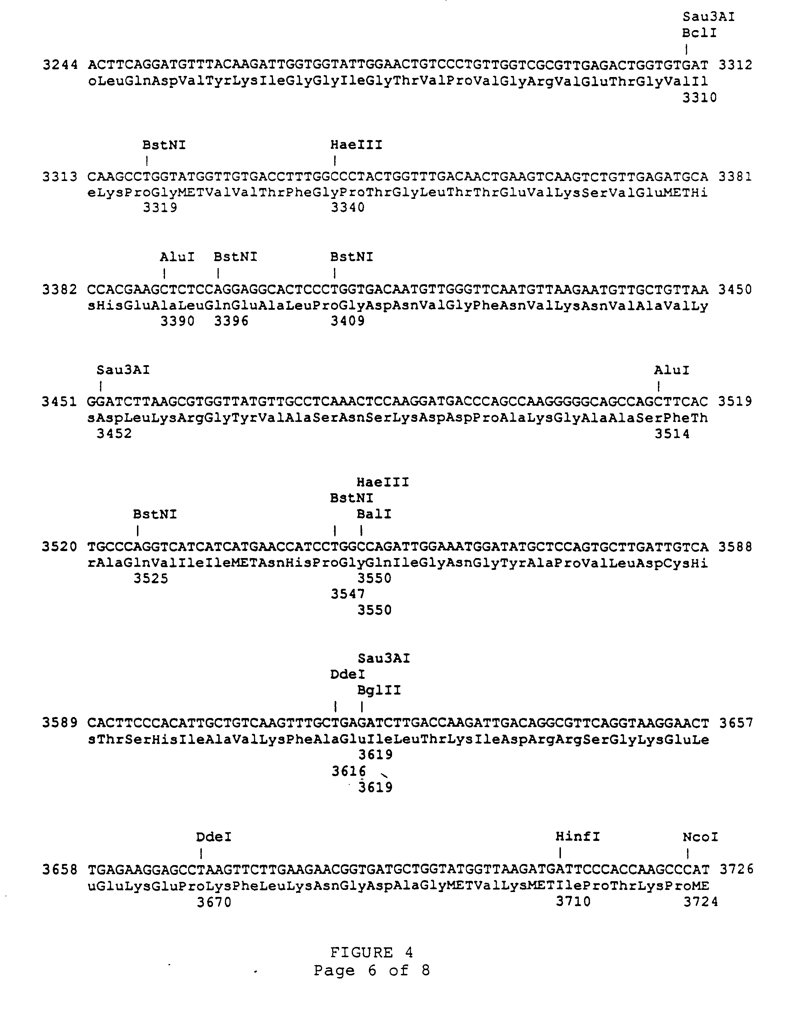

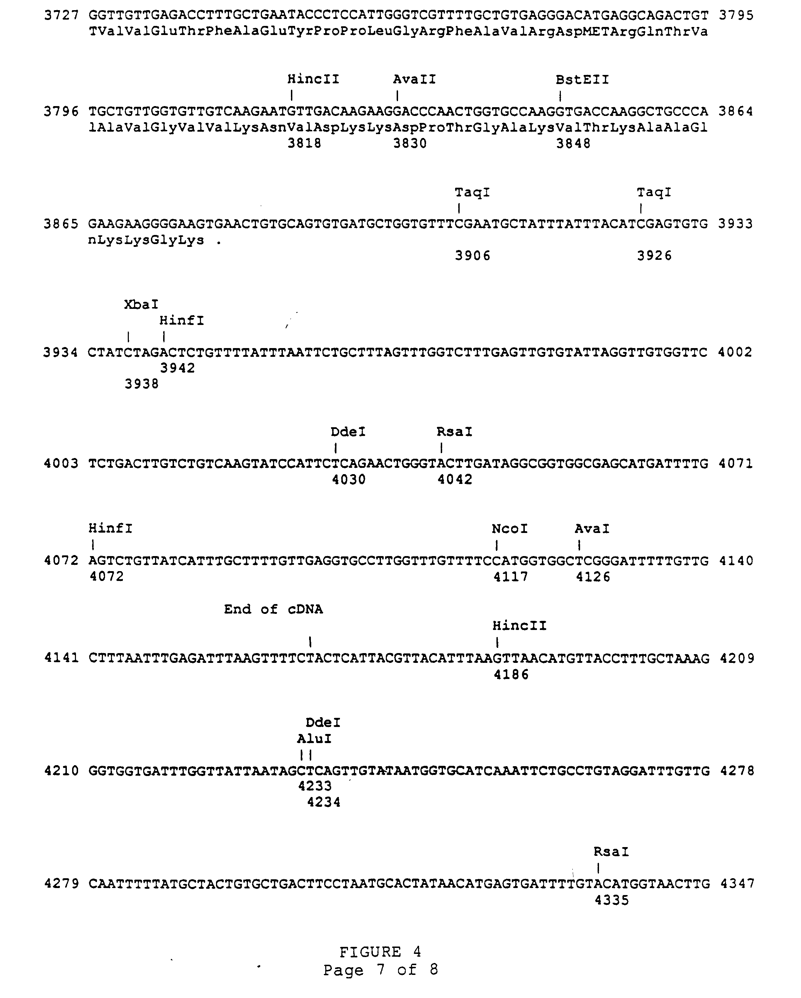

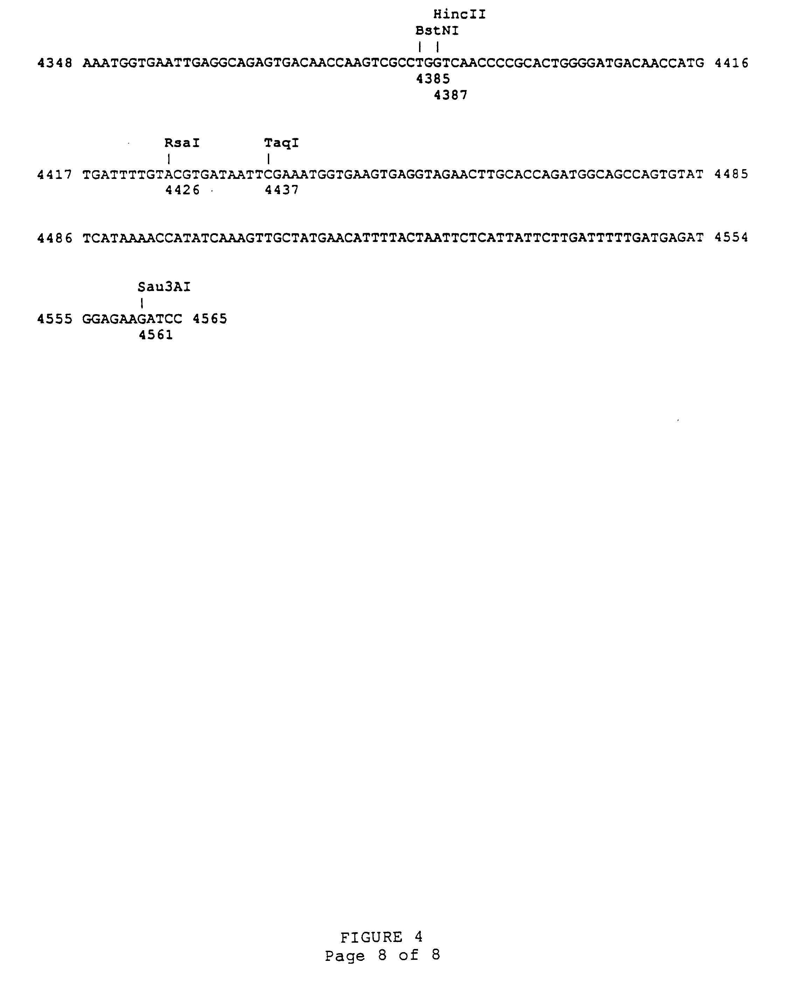

Novel DNA sequences, constructs employing such sequences, plant cells containing such constructs, and plants containing such constructs, are provided, where the sequences comprise the 5' non-coding region from the plant elongation factor-1α (EF-1α) sequences shown in Figure 3 and Figure 4. In addition, a method is described for identifying the genes for EF-1α, since it is found that EF-1α is a multi-gene family in plants. Plant EF-1α is characterized by having a mRNA of about 1.5-2 kb, a molecular weight in the range of about 45-55 kd, and a PI of about 8.5 to 9.5. The polypeptides encoded by plant EF-1αs have substantial homology with the polypeptides encoded by EF-1αs from a wide variety of species.

The transcriptional initiation regulatory region is found to provide regulation, where enhanced levels of EF-1α are expressed in rapidly dividing tissue, while lower levels are expressed in mature cells and tissue. An EF-1α gene isolated may be used to regulate expression in transgenic plants. For example, a promoter derived from an EF-1α gene has been shown to direct expression of a foreign gene in tomato and in tobacco plants.

Significant levels of plant EF-1α mRNA are found in substantially all tissues, e.g. young leaf, young root, mature leaf, mature root, green fruit, turning fruit and ripe fruit, generally ranging in values from 0.1 to 1.0%, as evidenced with the experience in tomato. Levels of EF-1α mRNA decrease in both the leaves and roots as they mature, and in fruit as it ripens.

The plant EF-1α sequence may be isolated from any convenient plant. When used in a construct, the EF-1α sequence may be endogenous to the target host or exogenous to the target host. Plants from which EF-1α may be isolated include fruit, vegetables, oil seeds, fiber sources, grasses, trees, flowers, grain, ornamentals and the like. Particular plants of interest include tomato, pea, tobacco, maize, soybean, Brassica, cotton, wheat, alfalfa, turf grass, and the like.

The plant EF-1α gene may be isolated by various techniques. These include isolating mRNA from a host organism which codes for the EF-1α, reverse transcribing the mRNA, and using the resulting single-stranded (ss) DNA as a template to prepare double-stranded (ds) DNA and the ds DNA isolated. Among multi-gene families it is desirable to find the transcriptional initiation regulatory region which provides a high level of transcription. Thus, the transcriptional initiation regulatory region should provide for at least about 10% of the total EF-1α mRNA, preferably at least about 20%, and more preferably at least about 30%. This can be determined by employing two probes, one probe which is conserved and binds to all EF-1α mRNA, and the other probe being in a polymorphic region of the EF-1α locus which binds uniquely to the EF-1α gene being assayed.

The sequences provided in the experimental section may be used to identify plant EF-1α genes from plant sources other than tomato. Particularly, by identifying sequences of the subject plant associated with the EF-1α gene, which sequences are conserved in species other than plants, these conserved sequences may be used as probes for hybridization to cDNA obtained from a number of different plant sources. Usually, the sequence will have at least about 60%, preferably at least about 70%, identity of base pairs, excluding any deletions which may be present. Thus cDNA libraries may be prepared from the plant source of interest, and the probes used to identify cDNA sequences for EF-1α. Conveniently, the target cDNA may be cloned in a virus, so that hybridizing phage may be plaque-purified. The identified cDNA's may be further sub-cloned and the sub-clone sequence analyzed and used for production of probes. The probes are then used to identify cDNA EF-1α sequences in a cDNA library. The cDNA is used to identify genomic sequences in a plant genomic library of the appropriate plant species and the positive clones analyzed by restriction enzyme digestion. The level of transcription may then be determined in a variety of plant tissues to demonstrate the pattern of transcription in the plant. In this manner, one or more sequences may be identified providing both the coding region, as well as the transcriptional regulatory elements of the gene.

The probes can be considerably shorter than the entire sequence, but should be at least about 10, preferably at least about 15, more preferably at least about 20 nucleotides in length. Longer oligonucleotides are also useful, up to the full length of the gene encoding the polypeptide of interest. Both DNA and RNA probes can be used.

In use, the probes are typically labeled in a detectable manner (for example with ³²P-labelled or biotinylated nucleotides) and are incubated with single-stranded DNA or RNA from the organism in which a gene is being sought. Hybridization is detected by means of the label after single-stranded and double-stranded (hybridized) DNA or DNA/RNA have been separated, typically using nitrocellulose paper. Hybridization techniques suitable for use with oligonucleotides are well known to those skilled in the art.

Although probes are normally used with a detectable label that allows for easy identification, unlabeled oligonucleotides are also useful, both as precursors of labeled probes and for use in methods that provide for direct detection of DNA or DNA/RNA. Accordingly, the term "oligonucleotide" refers to both labeled and unlabeled forms.

Once the 5'- and 3'-non-coding regions of the EF-1α gene have been identified, they may be manipulated in accordance with conventional ways. Where a convenient restriction site is present downstream from the ATG initiation codon, it may be useful to cut at that site and insert a DNA sequence of interest for transcription under the regulatory central of the EF-1α regulatory elements. Where the DNA sequence of interest is a structural gene, the sequence of interest will be inserted so as to be in reading frame with the upstream codons and the initiation methionine. Where the DNA is to be used as an anti-sense sequence, it need not be in reading frame with the upstream codons.

Usually, not more than 20 codons at the 5'-end of the EF-1α gene will be retained. Preferably, the sequence of interest will not be fused to the 5'-region of the EF-1α gene but rather may be joined to the transcriptional initiation regulatory region of EF-1α in a variety of ways. One can cleave internal to the EF-1α gene and resect using Bal31. By blunt-end ligation of the gene of interest to the various EF-1α fragments, one can screen for expression of the gene of interest indicating that a functional transcription initiation region has been retained. To join a sequence, one may use the polymerase chain reaction where an oligonucleotide containing the sequence of interest hybridizes at one end with the terminal sequence of the EF-1α transcriptional initiation regulatory region and/or 5'-untranslated region. By providing for replication using the polymerase chain reaction, dsDNA may be prepared for insertion into a vector which will have the EF-1α transcriptional initiation regulatory region properly joined to all of, or the 5'-proximal portion of, the sequence of interest.

Conveniently, one may identify a convenient restriction site in the 5'-untranslated region of EF-1α and in the 5' region of the gene of interest and employ an adapter which will join the two sequences and restore the lost sequences from the 5'-untranslated region and the 5' region of the gene of interest. Alternatively, one may introduce a polylinker immediately downstream from the 5'-untranslated region, for insertion of the DNA sequence of interest.

The 5'-non-coding region which will be employed for joining to the sequence of interest will usually be at least about 100 bp, and not more than about 10 kbp, frequently being less than about 2kbp, and may include all or a portion of an intron (including the splice sites). The 5'-non-coding region which is employed will be proximal to (usually within 20 bp) or abut the initiation codon. Therefore, by employing sequence analysis, one can identify the initiation codon of the EF-1α gene and isolate the upstream region in accordance with conventional ways. Where a particular cloned fragment in a genomic library does not have the desired size, the library may be further screened by walking the 5'-non-coding region until a fragment of the desired length is obtained. The 5'-non-coding region may then be cloned and sequenced and may then be used for further manipulation. As already indicated, in some instances, at least a fragment of the 5'-coding region may be retained.

The EF-1α transcriptional initiation regulatory region (promoter) may be inserted into a vector for preparing a construct for transcription and optionally translation. The 3' terminus of the EF-1α promoter may be joined to a sequence of interest, which may be a structural gene or coding or noncoding sequence where anti-sense may be desired. A wide variety of genes are of interest for transcription and optionally translation in rapidly dividing cells. For example, tender shoots and tissue are particularly sensitive to stress. Stress may take the form of toxins, such as pesticides, or herbicides, temperature changes such as heat or cold, including ice formation, osmotic pressure as a result of salinity, drought, and the like. Stress may include attack by various organisms or susceptibility to disease, as evidenced by insects, nematodes, aphids, caterpillars, fungi, and the like. Growth may be modulated, either increased or decreased, depending upon the particular need. Anti-sense sequences may be used to reduce growth, for example, altering the auxin/cytokinin ratio may be used to alter growth rate and/or morphology. By enhancing or diminishing the expression of an enzyme in the metabolic pathway for the hormone, the ratio may be modulated.

For resistance to toxins, various genes may be employed which may be the target of the toxin, where a mutated gene is employed which is less sensitive or insensitive to the toxin. Alternatively, one can enhance the amount of the target protein, so as to reduce the sensitivity of the plant cells to the toxin. For example, 5-enolpyruvyl-2-phosphoshikimate synthase is the target for glyphosate (aroA gene). By providing for an aroA gene which is less sensitive to the herbicide and/or enhancing the level of the enzyme present in the cell, particularly in the chloroplast, herbicide-resistance may be achieved. Alternatively, a gene which provides for detoxification may be employed, such as a nitrilase which hydrolyzes the nitrile group of bromoxynil. Additional examples are the herbicide phosphinothricin which may be detoxified by phosphinothricin acetyltransferase from Streptomyces hygroscopicus (bargene) and acetolactate synthase which may be mutated to be resistant to various sulfanyl-urea herbicides.

For insects or nematodes, there are naturally occurring toxins for the pest which may be provided as a gene for expression in the plant cells. For example a number of strains of Bacillus thuringiensis have relatively broad spectrum toxicity to a number of different insects as a result of expression of a protein toxin. By providing for expression of the protein toxin in plant cells, particularly among rapidly dividing cells, these cells will be protected from attack by such insects. Protection may also be provided against nematodes and fungus, and root worm, e.g. corn root worms.

For providing resistance to frost or heat, various genes may be employed, such as heat shock proteins, winter flounder antifreeze protein, etc. For stress tolerance such as salinity, Osm genes may be employed, such as genes associated with overproduction of proline. Additionally, stress tolerance may be provided by genes for superoxide dismutase.

The auxin/cytokinin ratio may be modified by modulating genes on the metabolic pathway to protect growing cells or to change growth rate. Other DNA sequences of interest in this regard include antisense EF-1α which may be used to modulate expression of EF-1α.

The EF-1α promoter, being a strong promoter, may be used in place of other strong promoters conventionally employed, such as the 35S cauliflower mosaic virus promoter. The subject promoters may also find use for a specific selectable marker where the target for transformation and regeneration is merisystematic tissue e.g., for use with particle bombardment in cotton, soybean, etc.

Depending upon the sequence of interest, the purpose of the transformation and the particular host, other sequences may be included which provide for specific functions. In some instances, it may be desirable to provide for translocation of the expression product from the cytoplasm to an organelle or for secretion. In this instance, various transit peptides may be employed for translocating the gene of interest to an organelle, such as the chloroplast or mitochondrion, or to secrete the protein into the extracellular space or to the cell surface. Various transit peptides have been employed, such as the transit peptide of the small subunit of the RUBISCO gene, plant EPSP synthase, acyl carrier protein, and the like.

In addition to the promoter and the structural gene, there will be a termination region, which may be the EF-1α termination region or any other convenient termination region. A wide variety of termination regions have been employed, such as termination regions from opine genes, various plant genes, and the like. The particular termination region will usually not be critical to this invention and any convenient region may be employed.

The promoter, structural gene and transcriptional and translational termination region provide an expression construct which may be joined to a vector for cloning. At each stage of the preparation of the construct, the resulting product may be cloned and analyzed to insure that the desired product has been obtained. Cloning vectors conveniently will have one or more markers, which will allow for detection of transformants which contain the construct and marker. For the most part markers will provide for toxin resistance or impart prototrophy to an auxotrophic host. Toxin resistance for the most part will be antibiotic resistance, such as resistance to kanamycin and its analogs, e.g. G418, resistance to chloramphenicol, and the like. For the most part cloning will be performed in E. coli, so that a replication system functional in E. coli will be employed.

Once the expression construct has been prepared and analyzed to insure it has the proper sequence, it may be then be used for introduction into a plant cell. Various techniques exist for introduction of DNA into plant cells. These techniques include A. tumefaciens mediated introduction, electroporation, protoplast fusion, injection, high velocity projectile introduction, and the like. The targets for introduction of the DNA may be tissue, particularly leaf tissue with A. tumefaciens, disseminated cells, protoplast, seeds, embryo, meristematic regions, cotyledons, hypocotyls, and the like.

With A. tumefaciens introduction, the construct will be further modified by having one or both T-DNA borders present, bordering the expression construct, particularly the right border. The construct may be introduced into A. tumefaciens carrying the vir genes, where the T-DNA bordered expression construct will be introduced into plant cells infected with A. tumefaciens. The plants which may be genetically modified include those plants described as the source of the EF-1α gene. Thus, of particular interest are those crops associated with food sources, such as grain, vegetables and fruits, oil seed, sugar sources, forage, and legumes.

Once the cells are transformed, transgenic cells may be selected by means of a marker associated with the expression construct. The expression construct will usually be joined with a marker which will allow for selection of transformed plant cells, as against those cells which are not transformed. The marker will usually provide resistance to an antibiotic, which antibiotic is toxic to plant cells at a moderate concentration.

After transformation, the plants cells may be grown in an appropriate medium. In the case of protoplasts the cell wall will be allowed to reform under appropriate osmotic conditions. In the case of seeds or embryos, an appropriate germination or callus initiation medium would be employed. For explants, an appropriate regeneration medium would be used.

The callus which results from cells may be introduced into a nutrient medium which provides for the formation of shoots and roots and the resulting plantlets planted and allowed to grow to seed. During the growth, tissue may be harvested and screened for the presence of expression of the expression construct. After growth, the seed may be collected and replanted, or prior to seed formation, the modified plant may be used for fertilizing a different strain or vice versa, so as to provide for a hybrid plant. One or more generations may then be grown to establish that the gene is inherited in Mendelian fashion.

Of particular interest is a gene associated with tomato, which has the amino acid and DNA sequence found in the Experimental section. The gene and its accompanying untranslated regions may be used in the anti-sense direction to inhibit expression in plant cells, so as to reduce growth. Thus, fragments of the gene and the untranslated regions of at least about 12 bp, usually at least about 20 bp may be employed to produce an mRNA which results in reduction in expression of the EF-1α gene.

The invention now being generally described, it will be more readily understood by reference to the following examples which are included for purposes of illustration only and are not intended to limit the invention unless so stated.

### EXPERIMENTAL

### Example I

### EF-1α cDNA

### A. Materials and Methods

### 1. Screening a Tomato cDNA Library for EF-1α Clones

A red, ripe tomato (Lycopersicon esculentum cv "Caligrande") cDNA library in λgt10 (Sheehy, et al., Mol. Gen. Genet. (1987) 208:30-36) was screened for EF-1α cDNAs. 1 x 10⁵ recombinant plaques of the λgt10 library grown in E. coli strain C600 hf1A150 (Hoyt, et al., Cell (1982) 31:565-573) were transferred to GeneScreen Plus filters (New England Nuclear) denatured, and neutralized by a modification of the procedure of Benton and Davis, Science (1977) 196:180-182, as described in O'Neal et al., Nucl. Acids Res. (1987) 15:8661-8667). The probe used was isolated from pMUEF-1 which contains the Mucor racemosa EF-1α gene, TEF-1 (Linz, et al., Mol. Cell. Biol. (1986) 6:593-600. A 471 bp ClaI fragment from pMUEF1-2, which spans nucleotides 219 to 689 of TEF-1 (corresponding to amino acids 73 to 230) (Linz, et al., (1986) Mol. Cell. Biol., 7:1925-1932) was isolated and nick-translated as previously described (Shewmaker, et al., Virology (1985) 140:281-288). Prehybridization, hybridization and washing of filters were done at a temperature of 33°C.

Ten separate plaques corresponding to ten different positive signals were picked, replated, and rehybridized until all were plaque purified. The inserts on these ten clones were sized by EcoRI digestion. A clone with an approximately 1.7 kb insert, designated LeEF-1, was chosen for further analysis. The EcoRI fragment of LeEF-1 was subcloned into pUC19 (Yanisch-Perron et al., Gene (1985) 33:103-119) yielding pCGN666. This EcoRI fragment was also cloned in both orientations into M13mp19 yielding pCGN667 and pCGN668, and into Bluescript M13+KS (Stratagene), yielding pCGN669 and pCGN670. These subclones were used for sequence analysis and generation of ³⁵S-RNA probes. The cDNA sequence of pCGN666 has been filed with the EMBO Gene Bank Data Library Postfach 10.2209, Meyerhofstrasse 1, 6900 Heidelberg, West Germany, on March 6, 1989. The file number is X14449.

### Sequencing Analysis

The cDNA in pCGN670 was sequenced in the 5' to 3' direction using ExoIII-S1 nuclease nested deletions (SacI, BamHI double digested) (these deletions were made using the Promega Biotec Erase A-Base kit according to manufacturer's instructions). The dideoxy method (Sanger, et al., Proc. Natl. Acad. Sci. USA (1977) 74:5463-5467 was used to sequence subclones from the deletion series. To overcome gaps not covered by the nested deletions, oligomers were synthesized (Applied Biosystems 380 A synthesizer, Foster City, CA) based on known sequences from the previously sequenced subclones. The oligomers served as primers for the pCGN667 template. For sequence confirmation, the EF-1α cDNA was also sequenced in the 3' to 5' direction using the Sanger method on pCGN668 utilizing synthetic oligomer primers. To sequence the region nearest the polyA⁺ tail in pCGN668, the Maxam-Gilbert method (Maxam and Gilbert, Proc. Natl. Acad. Sci. USA (1977) 74:560-564) was used, sequencing through the polyA⁺ tail and a small distance beyond, to overlap with the previously obtained sequence.

### Isolation of RNA and Northern Analysis

Tissue from various anatomical parts and developmental stages of tomato cultivar UC82B was frozen in liquid nitrogen until needed for analysis. From mature three and a half month old green house grown tomato plants (UC82B), the following tissue was taken: mature leaf, mature root, young leaf, green fruit, turning fruit, and red fruit (fruit ripening stages were determined from Color Classification Requirements in Tomato, USDA Visual Aid TM-:1; Feb. 1975, The John Henry Co., P.O. Box 17099, Lansing, MI). The entire compound leaf (stem included with leaflets) was harvested to isolate mature leaf RNA. Mature root RNA was isolated from the cleaned-up root ball. Young leaves were collected as axillary shoots (about five inches from base to top). Tissue from young root was collected from one and one-half week old germinated seedlings of UC82B. Seeds were surface sterilized in a 0.1% sodium hypochlorite, 0.1% Tween 20 (Sigma Chemical Co.) solution for 15 minutes, rinsed in sterile, distilled water and germinated on moistened autoclaved germinating paper (Anchor Paper Co., St. Paul, MN).

Tomato total RNA was prepared as described in Colbert, et al., Proc. Natl. Acad. Sci. USA (1983) 80:2248-2252 with a slight modification (Facciotti, et al., Bio/Technology (1985) 3:241-246). PolyA⁺ mRNA was isolated by oligodT cellulose column chromatography as described in Colbert, et al. (1983) supra; Maniatis, et al., Molecular Cloning: A Laboratory Manual, Cold Spring Harbor, New York (1982).

Northern analysis using nick-translated probes was performed as described (Facciotti, et al., supra). When olignoucleotides were used as probes for Northern analysis, the procedure was performed as described in O'Neal, et al., Nucl. Acids Res. (1987) 15:8661-8667, except that the final washes were with 0.5xSSC, 0.1% SDS at 45°C, for 2 x 15 min.

### DNA Isolation and Southern Analysis

Bacteriophage DNA was isolated from crude cell lysates with the Lambda Sorb (Promega Biotech) phage absorbent using the manufactuer's protocol. Tomato genomic DNA was isolated by the CTAB (cetyltrimethlammonium bromide) procedure (Taylor and Powell, Focus (1982) 4:4-6). Southern analysis was performed as described in Shewmaker, et al., (1985), supra.

### In vitro translation analysis

Poly(A⁺) mRNA was isolated from the fruit of cultivar "UC 82B" by the method of Sheehy, et al., Mol. Gene. Genet. (1987) 208:30-36. Approximately 0.5 µg of polyA⁺ RNA was translated in a reticulocyte lysate (Promega Biotech) as described by the manufacturer. ³⁵S-methionine-labelled translation products were precipitated with five volumes of acetone and collected by centrifugation. Samples were subjected to nonequilibrium two-dimensional gel electrophoresis as described by Garcia, et al., J. Bact. (1980) 142:196-201.

### ³⁵S-RNA Probe Generation

Sense and antisense ³⁵S-RNA probes were generated from pCGN669 and pCGN670 respectively using T3 polymerase an an in vitro transcription reaction. These plasmids were linearized at a unique BamHI site within the Bluescript polylinker. DNA was purified after BamHI digestion by extracting with phenol twice and phenol/chloroform/isoamyl alcohol (25:24:1) once. The extracted DNA was then ethanol precipitated and was resuspended in sterile water at a concentration of 2µg/µl. In an RNase-free microfuge tube, the following were mixed in the order given: 2µl 5X transcription buffer (200 mM Tris, pH7.5, 30 mM MgCl₂, 10 mM spermidine), 0.5 µl 100 mM DTT, 0.5 µl RNase inhibitor (RNasin) (25 U/µl, Promega Biotech), 2µl 10X rNTPs (2,5 mM GTP, 2.5 mM ATP, 2.5 mM CTP, 75 µM UTP), 4.5 µl α-³⁵S-UTP (10 µCi/µl, New England Nuclear), 0.5 µl DNA (2 µg/µl), and 0.5 µl T3 polymerase (50 U/µl, Stratagene). After careful mixing, the reaction mixture was incubated at 37°C for one hour. After one hour, 0.5 µl RNasin (25 U/µl, Promega Biotech) and 0.5 µl RQ1 DNAse (1 U/µl, Promega Biotech) were added and the reaction incubated for an additional 10 minutes. The reaction was stopped by the addition of 1 µl 250 mM EDTA, pH 7.0. Unincorporated ribonucleotide triphosphates were separated from RNA transcripts by Sephadex G-75 gel filtration chromotagraphy. Both pCGN669 and pCGN670 had labeled peaks of approximately 4.0 X 10⁸ Cpm/µg RNA.

### In situ RNA Hybridization

Root tips from one and one-half week old tomato seedlings were fixed in a 4% paraformaldehyde/phosphate buffered saline (PBS)/5mM MgCl₂ solution, pH 7.4 (PBS is 10 mM pH 7.4 phosphate buffer, made to 150 mM NaCl) (Singer, et al., Biotechdniques (1985) 4:230-250). Tissue was fixed overnight. After fixation, the tissue was passed through a graded tertiary butyl alcohol (TBA) series starting at 50% TBA, infiltrated with Paraplast and cast into paraffin blocks for sectioning, (Berlyn and Miksche (1976) Botancial Microtechnique and Cytochemistry, State University Press, Ames, Iowa). Embedded root tips were sectioned longitudinally on a Reichert Histostat rotary microtome at 4 µM to achieve a one-cell layer thickness. Paraffin ribbons holding 7-10 root tip sections were affixed to gelatin-chrom alum subbed slides (Berlyn and Miksche (1976), supra) and held in a dust-free box until in situ hybridizations were performed. Slides ready to be hybridized were deparaffinized in xylene and rehydrated by passing through an ethanol hydration series as described in Singer, et al., Biotechniques (1986) 4:230-250.

A 2X hybridization mix was made consisting of 100 µl 20X SSC, 20 µl 10% BSA, 100 µl 750 mM DDT, 200 µl 50% dextran sulfate, 50 µl RNasin, and 30 µl sterile water. 2.5 µl tRNA (20 mg/ml), 2.5 µl salmon sperm DNA (10 mg per ml) and 4 X 10⁶ cpm probe were dried down on a lyophilizer. This mix was then resuspended in 25 µl 90°C formamide and 25 µl 2X hybridization mix per slide. 40 µl of this hybridization mix was placed on each slide. A cover slip was placed over the sections and edges sealed with rubber cement. Slides were placed in slide holders inside a glass slide box, covered, and placed in a 37°C dry oven overnight to hybridize. Posthybridization treatments were as described in Singer, et al., (1986), supra.

Autoradiography was performed as described in Kodak Materials for Light Microscope (1986) (available from Kodak), using Kodak liquid emulsion NTB-3. Slides were exposed in a light box for approximately two weeks. After developing the autoradiographic slides, root sections were stained in 0.05% toluidine blue and then dehydrated through a graded alcohol series: xylene: 100% ethanol, 1:1, followed by 2 changes 100% xylene. Slides were left for five minutes in each solution. Coverslips were mounted with cytoseal (VWR) and left on a slide warmer until dry (45-50°C, 1-2 days). Autoradiographic slides were then ready for microscopic examination and photomicrography.

### B. Results

To isolate a tomato EF-1α cDNA clone, a fragment from a Mucor racemosa EF-1α cDNA clone was used (Linz and Sypherd, Mol. Cell Biol. (1987) 7:1925-1932). Preliminary analysis using this probe to tomato RNA indicated that under the conditions used, the Mucor probe hybridized solely to a bind of approximately 1.8kb. This agrees with the size reported for other EF-1α cDNA RNA's (Nagata, et al., EMBO J. (1984) 3:1825-1830; Linz et al., Mol. Cell Biol. (1986) 6:593-600), and is within the expected size range of the protein (45-55kd).

The clone containing the largest insert of 1.7kb, hereinafter termed LeEF-1, was chosen for further analysis.

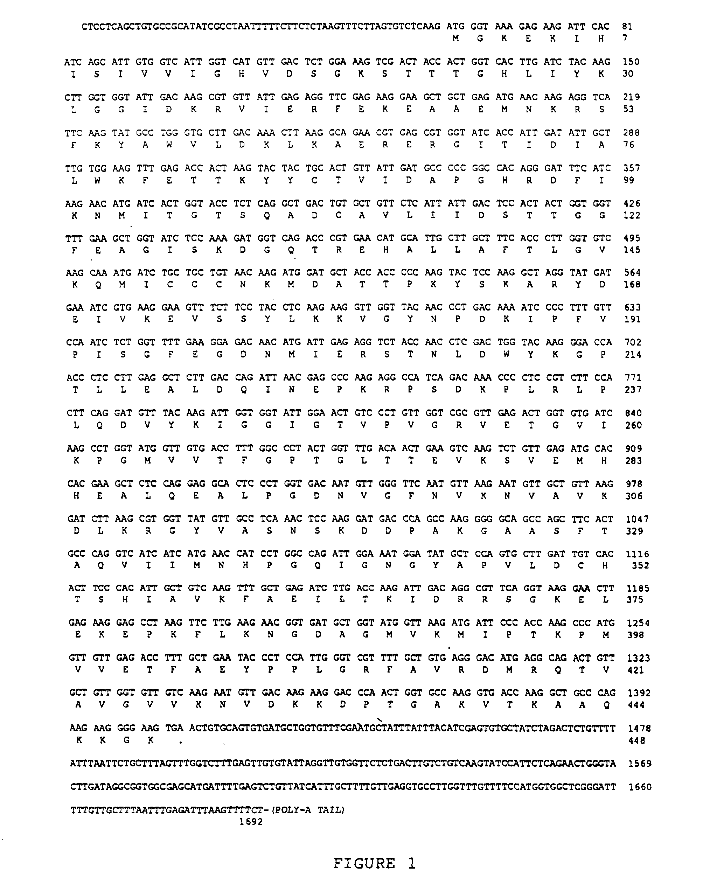

LeEF-1 contains 60 nucleotides upstream of the ATG, a 1344 nucleotide (448 amino acids) coding region and 284 nucleotides of 3'-untranslated region. In the 5'-untranslated region there are two in-frame stop codons. In-frame stop codons are also found in the 5' untranslated regions of other EF-1α genes. In the LeEF-1, an adenine residue is at the -3 position and a guanine residue is at the +4 position relative to the start AUG codon. This nucleic acid sequence surrounding the initiator codon displays the consensus signal for efficient translation initiation. The complete sequence of LeEF-1 is shown in Figure 1.

Amino acid sequence comparisons between the polypeptides encoded by tomato EF-1α gene, LeEF-1 and an EF-1α gene from Mucor, as well as Artemia, yeast, and man show substantial homology. The LeEF-1 gene product is 76% homologous to that of yeast and Mucor; 75% homologous to that of Artemia; and 78% homologous to that of man.

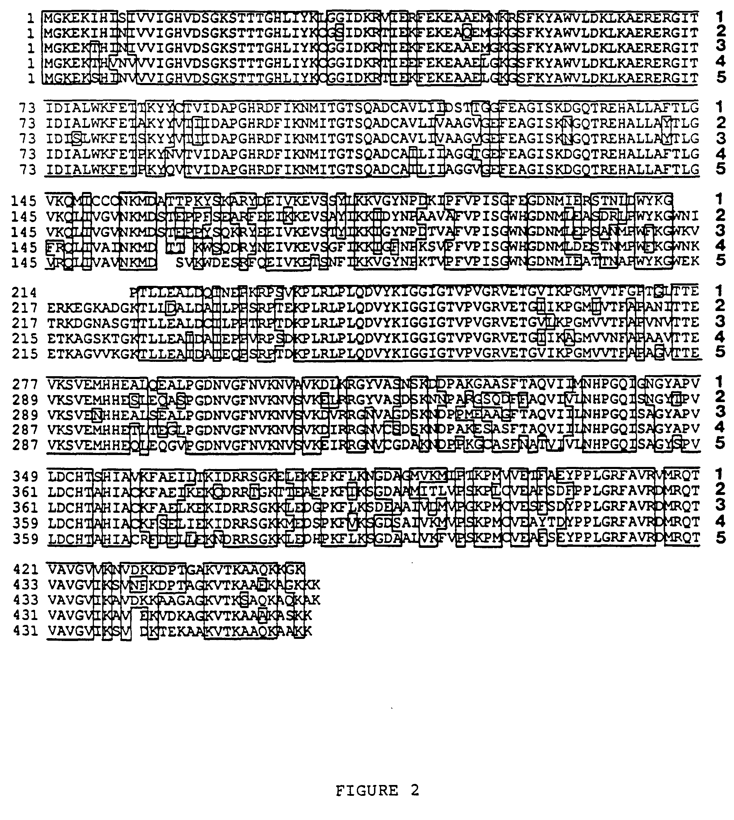

As shown in Figure 2, one notable difference between LeEF-1 and the four other eukaryotic EF-1α's is that a 12 amino acid gap must be introduced between amino acids 213 and 214 of the protein encoded by LeEF-1 to provide maximum homology to the other eukaryotic EF-1α gene products. The homology between the polypeptides encoded by the four other eukaryotic EF-1α genes in this gap region ranges from 29% to 64% and no specific function has been previously assigned to this region.

### EF-1α Is A Multigene Family In Tomato

To determine if EF-1α in tomato was encoded by one or multiple genes, Southern blot analysis was performed. When tomato DNA restricted with EcoRI was hybridized to LeEF-1, at least six distinct bands were seen. This indicated that EF-1α is probably a multigene family. This was verified by screening a tomato genomic library for EF-1α clones. Six distinct clones obtained from this screen were restricted with EcoRI and hybridized to LeEF-1. These six distinct clones represent four of the bands seen on the genomic Southern blot. The EcoRI fragment from one of these clones (61-1) was subcloned and shown to contain an entire EF-1α gene (see Example II). While it is clear that EF-1α in tomato is a multigene family, the exact size of the family remains to be determined.

### EF-1α Is Expressed In Rapidly Growing Tissue

Various tomato mRNA's were run on a Northern gel and hybridized against the tomato EF-1α cDNA. Specifically, the probe was the 1.7 kb EcoRI fragment from pCGN666 which had been labelled by nick-translation. Tissues chosen for analysis were young leaf, young root, mature leaf, mature root, green tomato fruit, turning tomato fruit, and red ripe tomato fruit. Significant levels of EF-1α mRNA were found in all tissues examined with values ranging from 0.1 to 1.0% of total mRNA. Levels of EF-1α mRNA decreased in both the leaves and roots as they matured and in the fruit as it ripened.

The expression pattern of EF-1α was further examined *in vitro* translation. Poly(A⁺) RNA from green and red (mature) tomato fruit was isolated, translated and subjected to 2-D gen analysis. The EF-1α protein is easily identifiable because of its molecular weight (50,000 daltons) and its high pI of around 9. While EF-1α protein is produced from mRNA of both green and red fruit, more is produced by the RNA from younger green fruit. These data, taken together with the Northern analysis, indicate that EF-1α is a highly expressed protein in tomato and that this expression is greater in young, rapidly growing tissue than in older, more mature tissue.

### 5. Localization of EF-1α, RNA To Meristematic Regions of The Root Tip

To further characterize the expression pattern of EF-1α, *in situ* hybridizations were performed. In root tips there are well-defined meristematic regions that are associated with active cell division as well as regions associated with cell elongation (Raven, et al., Biology of Plants (1976); Essau, Anatomy of Seed Plants (1977); Galston, et al., The Life of The Green Plant (1980)). When 4-mm root tips from young tomato plants were hybridized to sense and antisense ³⁵S-EF-1α RNA, the antisense transcripts hybridized to distinct regions of the root tips, while the sense transcripts (negative control) showed no hybridization. More hybridization was seen in the first 1 to 2 mm of the root tip, which corresponds to the region of cell division, than in the tissue further up the root tip, known as the region of cell elongation. Strong, defined hybridization was seen in the three primary meristems: protoderm, ground meristem, and procambrium, as well as in the apical meristem. The three meristems in a root give rise to the epidermis, cortex and primary vascular tissue respectively. Thus, within a root tip, the highest levels of EF-1α mRNA were found in meristematic or rapidly dividing cells. In summation, these in situ results corroborated the Northern data to demonstrate that EF-1α mRNA in tomato is particularly abundant in actively dividing cells.

### Example II

### ISOLATION AND CHARACTERIZATION OF AN EF-1α GENOMIC CLONE 61-1

### A. Materials and Methods

### 1. Screening a tomato genomic library for EF-1α genomic clones.

A tomato (Lycopersicon esculentum cv 'T6') partial EcoRI genomic library was provided by Dr. Robert Goldberg, UCLA. This library was screened for EF-1α genes using the nick-translated tomato cDNA, LeEF-1 as a probe. Approximately eight tomato genome equivalents in 800,000 plaques of the Charon4 phage in E. coli host LE392 (F⁻ hsdR514 supE44 supF58 1acY1 or lac(1-Y6 galK2 galT22 metB1 trpR55 9λ⁻) were screened. DNA was transferred to Gene Screen Plus filters as described in Example I for screening the tomato cDNA. Prehybridization, hybridization, and washing of filters were as described in Shewmaker, et al. (1985), supra). Filters were prehybridized and hybridized at 42°C and washed at 55°C. A number of positive plaques were picked and replated until plaque purified. The tomato inserts in positive clones were sized by EcoRI digestion.

### 2. Isolation, mapping and sequencing of an EF-1α genomic clone (61-1) from tomato.

### a. Construction of pCGN2123

One EF-1α lambda clone (61-1) containing a 5.6 kb EcoRI fragment that hybridized to LeEF-1 was chosen for further analysis. DNA from 61-1 was prepared using a Lambda Sorb kit (Promega Biotech) according to manufacturer's instructions. The 5.6 kb EcoRI piece was subcloned into pUC19 yielding pCGN2123. Using standard mapping techniques (Maniatis, et al., (1982) Molecular Cloning, A Laboratory Manual, Cold Spring Harbor, N.Y.), the approximately 2 kb EF-1α gene was located within the 5.6 kb fragment. The coding region plus approximately 800 bp 5' of the start codon and approximately 1200 bp 3' of the stop codon were sequenced. Sequence analysis was performed using standard techniques, similar to those described for the sequencing of LeEF-1.

### 3. Construction of Binary Plasmid pCGN783

pCGN783 is a binary plasmid containing the left and right T-DNA borders of A. tumefaciens (Currier and Nester, J. Bacterial, (1976), 126:157-165); the gentamicin resistance gene of pPH1JI (Hirsch, et al., Plasmid (1984) 12:139-141); the 35S promoter of cauliflower mosaic virus (CaMV) (Gardner, et al., Nucleic Acids Res. (1981) 9:2871-2888); the kanamycin resistance gene of Tn5 (Jorgenson et al., Mol. Gen. Genet., (1979) 177:65-); and the 3' region from transcript 7 of pTiA6 (Currier and Nester (1976), supra). pCGN783 was constructed by the multistep process described below. pCGN783 has been deposited with the American Type Culture Collection (ATCC 67868).

### Construction of pCGN451

pCGN451 contains the ocs5'-ocs3' cassette cloned into a derivative of pUC8 (Vieira and Messing, Gene, (1982), 19:259-268). The modified vector was derived by digesting pUC8 with HincII and ligating in the presence synthetic linker DNA, creating pCGN416, and then deleting the EcoRI site of pCGN416 by EcoRI digestion followed by treatment with Klenow enzyme and self ligation to create pCGN426.

The ocs5'-ocs3' cassette was created by a series of steps from DNA derived from the octapine Ti-plasmid pTiA6 (Currier and Nester, J. Bact. (1976) 126:157-165). An EcoRI fragment of pTiA6 (bp 13362-16202; the numbering is by Barker, et al., Plant mol. Biol. (1983) 2:335-350), for the closely related Ti plasmid pTi15955) was removed from pVK232 (Knauf and Nester, Plasmid (1982) 8:45) by EcoRI digestion and cloned into EcoRI digested pACYC184 (Chang and Cohen, J.Bacteriol. (1978) 134:1141-1156) to generate pCGN15.

The 2.4 kb BamHI-EcoRI fragment (bp 13774-16202) of pCGN15 was cloned into EcoRI-BamHI digested pBR322 (Bolivar, et al., Gene (1977) 2:95-113) to yield pCGN429. The 412 bp EcoRI-BamHI fragment (bp13362-13774) of pCGN15 was cloned into EcoRI-BamHI digested pBR3322 (Bolivar, et al., Gene (1977) 2:95-113) to yield pCGN407. The cut-down promoter fragment was obtained by digesting pCGN407 with XmnI (bp 13512), followed by resection with Bal31 exonuclease, ligation of synthetic EcoRI linkers, and digestion with BamHI. Resulting fragments of approximately 130 bp were gel purified and cloned into M13mp9 (Vieira and Messing, Gene (1982) 19:259-268 and sequenced. A clone, I-4, in which the EcoRI linker had been inserted at bp 13642 between the transcription initiation point and the translation initiation codon was identified by comparison with the sequence of de Greve et al (de Greve, et al., J. Mol. Appl. Genet. (1982) 1:499-512). The EcoRI cleavage site was at position 13639, downstream from the mRNA start site. The 141 by EcoRI-BamHI fragment of 1-4, containing the cut-down promoter, was cloned into EcoRI-BamHI digested pBR322 (Bolivar, et al., Gene (1977) 2:95-113) to create pCGN428. The 141 bp EcoRI-BamHI promoter piece from pCGN428, and the 2.5 kb EcoRI-BamHI ocs 5' piece from pCGN429 were cloned together into EcoRI digested pUC9 (Vieira and Messing, Gene (1982) 19:259-268) to generate pCGN442, reconstructing the ocs upstream region with a cut-down promoter section.

The HindIII fragment of pLB41 (D. Figurski) containing the gentamicin resistance gene was cloned into HindIII digested pACYC184 (Chang and Cohen, J. Bacteriol. (1978) 134:1141-1156) to create pCGN413b. The 4.7 kb BamHI fragment of pTiA6 (Currier and Nester, J. Bact. (1976) 126:157-165) containing the ocs 3' region, was cloned into BamHI digested pBR325 (F. bolivar, Gene (1978) 4:121-136) to create 33c-19. The SmaI site a position 11207 of 33c-19 was converted to an XhoI site using a synthetic XhoI linker, generating pCGN401.2. The 3.8 kb BamHI-EcoRI fragment of pCGN401.2 was cloned into BamHI-EcoRI digested pCGN413b to create pCGN419.

The ocs5'-ocs3' cassette was generated by cloning the 2.64 kb EcoRI fragment of pCGN442, containing the 5' region, into EcoRI digested pCGN419 to create pCGN446. The 3.1 kb XhoI fragment of pCGN446, having the ocs 5' region (bp 13639-15208) and ocs 3' region (bp 11207-12823), was cloned into the XhoI site of pCGN426 to create pCGN451.

### Construction of pCGN587

pCGN587 was prepared as follows: The HindIII-SmaI fragment of Tn5 containing the entire structural gene for APH3'II (Jorgensen, et al., Mol. Gen. Genet. (1979) 177:65-) was cloned into HindIII-SmaI digested pUC8 (Vieira and Messing, Gene (1982) 12:259-268). This converted the fragment into a HindIII-EcoRI fragment, since there was an EcoRI site immediately adjacent to the SmaI site. The PstI-EcoRI fragment of pCGN300, containing the 3'-portion of the APH3'II gene, was then combined with an EcoRI-BamHI-SalI-PstI linker into the EcoRI site of pUC7 to make pCGN546W. An ATG codon was upstream from and out of reading frame with the ATG initiation codon of APH3'II. The undersired ATG was avoided by inserting a Sau3A-PstI fragment from the 5'-end of APH3'II, which fragment lacks the superfluous ATG, into the BamHI-PstI site of pCGN546W to provide plasmid pCGN550. The EcoRI fragment of pCGN550 containing the APH3'II gene was then cloned into the EcoRI site of pUC8-pUC13-cm (K. Buckley, Ph.D. Thesis, UC San Diego, (1985)) to give pCGN551. The plasmid pCGN451 having the ocs 5' and ocs 3' in the proper orientation was digested with EcoRI and the EcoRI fragment from pCGN551 containing the intact kanamycin resistance gene inserted into the EcoRI site to provide pCGN552 having the kanamycin resistance gene in the proper orientation. This ocs/KAN gene was used to provide a selectable marker for the trans type binary vector pCGN587.

The 5' portion of the engineered octopine synthase promoter cassette consisted of pTiA6 DNA from the XhoI site at bp 15208-13644 (Barker, et al., Plant Mol. Biol. (1983) 2:335-350), and also contains the T-DNA boundary sequence (border) implicated in T-DNA transfer. In the plasmid pCGN587, the ocs/KAN gene from pCGN552 provided a selectable marker as well as the right border. The left boundary region was first cloned in M13mp9 as a HindIII-SmaI piece (pCGN502) base pairs 602-2212 and recloned as a KpnI-EcoRI fragment in pCGN565 (a cloning vector containing vector based upon pUC8-cm but containing pUC18 linkers) to provide pCGN580. pCGN565 is a cloning vector based on a pUC8-pUC13-cm (K. Buckley (1985), supra) but containing pUC18 linkers (Yanisch-Perron, et al., Gene (1985) 33:103-119) pCGN580 was linearized with BamHI and used to replace the smaller BglII fragment of pVCK102 (Knauf and Nester, Plasmid (1982) 8:45), creating pCGN585. By replacing the smaller SalI fragment of pCGN585 with the XhoI fragment from pCGN552 containing the ocs/KAN gene, pCGN587 was obtained.

### Construction of pCGN739 (Binary Vector)

To obtain the gentamicin resistance marker, the gentamicin-resistance gene was isolated from a 3.1 kb EcoRI-PstI fragment of pPHIJ1 (Hirsch, et al., (1984), supra) and cloned into pUC9 (Vieira and Messing, Gene (1982) 19:259-268) yielding pCGN549. The HindIII-BamHI fragment from pCGN549 containing the gentamicin-resistance gene replaced the HindIII-BglII fragment of pCGN587, creating pCGN594. The pCGN594 HindIII-BamHI region, which contains a 5'-ocs-kanamycin-ocs-3' (ocs is octopine synthase with 5' designating the promoter region and 3' the terminator region, see co-pending U.S. application serial no. 834,161, filed February 26, 1986) fragment was replaced with the HindIII-BamHI polylinker region from pUC18 (Yanish-Peron, et al, Gene (1985) 33:103-119) to make pCGN739.

### Construction of pCGN734

The HindIII-SphI fragment of pTiA6 corresponding to bases 3390-3241 (Barker, et al., Plant Mol. Biol. (1983) 2:335-350) was cloned into the HindIII-SphI site of M13mp19 (Yanisch-Perron, et al., Gene (1985) 53:103-119; Norrander, et al., Gene (1983) 26:101-106). Using an oligonucleotide corresponding to bases 3287 to 3300, DNA synthesis was primed from this template. Following S1 nuclease treatment and HindIII digestion, the resulting fragment was cloned in the HindIII-SmaI site of pUC19 (Yanisch-Perron, et al., Gene (1985) 33:103-119). (This resulted in an intermediate plasmid that should be named?) An EcoRI-HindIII fragment corresponding to bases 3287-3390 (Barker, et al., Plant mol. Biol. (1983) 2:335-350) was cloned with the EcoRI-HindIII fragment of pTiA6 (corresponding to bases 3390-4494) into the EcoRI site of pUC8 Vieira and Messing, Gene (1982) 19:259-268) resulting in pCGN734.

### Construction of pCGN726c

pCGN556 contains the EcoRI-HindIII linker of pUC18 (Yanisch-Perron, et al., Gene (1985) 33:103-119) inserted into the EcoRI-HindIII sites of pUC13-cm (K. Buckley (1985), supra). The HindIII-BglII fragment of pNW31c-8,29-1 (Thomashow, et al., Cell (1980) 19:729-739) containing ORF1 and 2 of pTiA6 (Barker, et al., Plant Mol. Biol. (1983) 2:335-350) was subcloned into the HindIII-BamHI sites of pCGN556 producing pCGN703.

The Sau3A fragment of pCGN703 containing the 3' region of transcript 7 (corresponding to bases 2396-2920 of pTiA6 (Barker, et al., (1983) sutra) was subcloned into the BamHI site of pUC18 producing pCGN709. The EcoRI-SmaI polylinker region of pCGN709 was replaced with the EcoRI-SmaI fragment of pCGN587, which contains the kanamycin resistance gene (APH3-II) producing pCGN726.

The EcoRI-Sal1 fragment of pCGN726 plus the BglII-EcoRI fragment of pCGN734 were inserted into the BamHI-SalI site of pUC8-pUC13-cm (chloramphenicol resistant, K. Buckely, (1985), supra) producing pCGN738. pCGN726c was derived from pCGN738 by deleting the 900 bp EcoRI-EcoRI fragment.

### Construction of pCGN528 (Shuttle Vector)

pCGN525 was made by digesting a plasmid containing Tn5 which harbors a kanamycin gene (Jorgenson, et al., Mol. Gen. Genet. (1979) 177:65) with HindIII-BamHI and inserting the HindIII-BamHI fragment containing the kanamycin gene into the HindIII-BamHI sites into the tetracycline gene of pACYC184 (Chang and Cohen, J. Bacteriol. (1978) 134:1141-1156). pCGN526 was made by inserting the BamHI fragment 19 of pTiA6 (Thomashow, et al., Cell (1980) 19:729-739), modified with XhoI linkers inserted into the SmaI site, into the BamHI site of pCGN525. pCGN528 was obtained by deleting the small XhoI fragment from pCGN526 by digesting with XhoI and religating.

### Construction of pCGN167

The AluI fragment of CaMV (bp 7144-7735) (Gardner, et al., Nucl. Acid Res. (1981) 9:2871-2888) was obtained by digestion with AluI. It was then cloned into the HincII site of M13mp7 (Messing, et al., Nucl. Acids Res. (1981) 9:309-321) to create C614. An EcoRI digest of C614 produced the EcoRI fragment from C614 containing the 35S promoter which was cloned into the EcoRI site of pUC8 (Vieira and Messing, Gene (1982) 19:259-268) to produce pCGN146. To trim the promoter region, the BalII site (bp 7670) was treated with BglII and resected with Bal31. Subsequently a BalII linker was attached to the Bal31 treated DNA to produce pCGN147.

pCGN148a, containing a promoter region, selectable marker (KAN with 2 ATG's) and 3' region, was prepared by digesting pCGN528 with BglII and inserting the BamHI-BglII promoter fragment from pCGN147. This fragment was cloned into the BglII site of pCGN528 so that the BglII site was proximal to the kanamycin gene of pCGN528.

pCGN149a was made by cloning the BamHI-kanamycin gene fragment from pMB9KanXXI into the BamHI site of pCGN148a. pMB9KanXXI is a pUC4 variant (Vieira and Messing, Gene (1982) 19:259-268) which has the XhoI site missing but contains a functional kanamycin gene from Tn903 to allow for efficient selection in Agrobacterium. pCGN149a was digested with BglII and SphI. This small BglII-SphI fragment of pCGN149a was replaced with the BamHI-SphI fragment from MI (see below) isolated by digestion with BamHI and SphI. This produces pCGN167, a construct containing a full length CaMV promoter., 1ATG-kanamycin gene, 3' end and the bacterial TN903-type kanamycin gene. MI is an EcoRI fragment from pCGN550 (see construction of pCGN587) and was cloned into the EcoRI cloning site of M13mp9 so that the PstI site in the 1ATG-kanamycin gene was proximal to the polylinker region of M13mp9.

### Construction of pCGN766c (35S promoter-3' region)

The HindIII-BamHI fragment of pCGN167 containing the CaMV-35S promoter, 1ATG-kanamycin gene and the BamHI-fragment 19 of pTiA6 was cloned into the BamHI-HindIII sites of pUC19 (Yanisch-Perron (1985), supra) creating pCGN976. The 35S promoter and 3' region from transcript 7 was developed by inserting a 0.7 kb HindIII-EcoRI fragment of pCGN976 (35S promoter) and the 0.5 kb EcoRI-SauI fragment of pCGN709 (transcript 7:3') into the HindIII-SalI sites of pCGN566 (pCGN566 contains the EcoRI-HindIII linker of pUC18 inserted into the EcoRI-HindIII sites of pUC13-Cm) creating pCGN766c.

### Final Construction of pCGN783

The 0.7 kb HindIII-EroRI fragment of pCGN766c (CaMV-35S promoter) was ligated to the 1.5 kb EcoRI-SalI fragment of pCGN726c (1ATG-Kan 3' region) followed by insertion into the HindIII-SalI fragment of pCGN778 containing the CaMV-35S promoter and 1ATG-KAN-3' region was used to replace the HindIII-SalI linker region of pCGN739 to produce pCGN783.

### 4. Construction of pCGN2125, an EF-1α GUS Fusion Construct

To analyze the expression of 61-1 when reintroduced into tomato plants, the GUS (β-glucuronidase, E. coli) gene was inserted into the coding region of 61-1. The fusion was done so that the GUS coding region was in-frame with the EF-1α sequence so that a protein with GUS activity could be produced.

### a. Construction of pCGN2125

To construct pCGN1804, the approximately 2 kb BamHI-SstI fragment from pBI221 containing the GUS gene (Jefferson, Plant Mol. Biol. Reporter (1987) 5:387-405) was cloned into pUC119. To construct pCGN2124, pCGN2123 (see Example II A.2.a.) was linearized with ClaI, and treated with the Klenow fragment of DNA polymerase I to blunt the ends. The resulting DNA was ligated to the BamHI-EcoRI fragment of pCGN1804, containing the GUS gene, which had also been treated with the Klenow fragment of DNA polymerase. A clone, pCGN2124, was chosen that had this fragment inserted in the correct orientation. The ClaI/BamHI junction between EF-1α and GUS was sequenced to verify that the GUS insertion was in-frame with the EF-1α coding region. The fusion contained the first 34 amino acids of EF-1α from 61-1 and 8 amino acids of linker region followed by the GUS gene.

To facilitate transfer to tomato plants, pCGN2124 was inserted into the Agrobacterium tumefaciens binary vector, pCGN783 (see above). pCGN2124 was linearized with SalI and inserted into the SalI site of pCGN783 (supra). A clone, pCGN2125, with the EF-1α GUS gene being transcribed in the same direction as the 35S-kan-tml gene was obtained. This construct in the Agrobacterium tumefaciens LBA4404 (also referred to as 2760) was used to transform tomato plants.

### 5. Construction of pCGN7001 (mas-gus-mas construct)

To evaluate levels of GUS produced from pCGN2125 in tomato plants, tomato plants containing a mas-gus-mas construct (pCGN7001) were generated. The mas (mannopine synthase promoter) has been previously studied and shown to give a relatively low level of expression (Murai and Kemp, Nucl. Acids Res. (1982) 10:1679-1689).

To construct pCGN7001, a 5' mas-gus-mas 3' sequence was inserted into the Sail site of pCGN783. The 5' mas-gus-mas 3' construct contained sequentially the following sequences: Xho linker; the NaeI to blunted ClaI fragment of the T-DNA of pTiA6 which spans the nucleotides 20806 to 20128 (the numbering is by Barker, et al., Plant Mol. Biol. (1983) 2:335-350 for the closely related Ti plasmid pTi 15955); the AccI to BamHI linker region of pUC19; the BamHI to SstI fragment of pCGN1804 containing the β-glucuronidase gene; the SstI to EcoRI linker region of pUC19; EcoRI linker; the blunted HindIII to blunted SstI fragment of pTiA6 which spans the nucleotides 19239 to 18462; (Barker, et al., supra); Xho linker.

The 5' mas-gus-mas 3' sequence was inserted into pCGN783 so that the 5' mas-gus-mas 3' sequence was transcribed in the same direction as the 35S-kan-tml gene. This construct in the Agrobacterium tumefaciens LBA4404 was used to transform tomato plants.

### 6. Transformation of Tomato Cotyledon Tissue

### a. Preparation of A. tumefaciens LBA 4404/pCGN7001 and pCGN2125

The plasmids pCGN7001 or pCGN2125, respectively, were transformed into E. coli C2110 (polA) as follows.

Bacterial matings were performed using two E. coli strains and Agrobacterium tumefaciens strain LBA4404. One E. coli strain (MM294) harbored pRK2073 which provided mobilization functions and the other strain (C2110) carried the plasmid with a kanamycin resistance marker to be transferred into Agrobacterium. The two E. coli strains were grown overnight at 37°C with shaking in LB broth. The Agrobacterium strain was grown overnight at 28°C in MG/L broth. Fifty microliters of each of the three strains were mixed on a nitrocellulose filter placed on an MG/L plate. The plate was incubated at 28C for 3 days. The mixture was then streaked onto an AB minimal medium supplemented with 100µg/ml kanamycin and 100µg/ml streptomycin and incubated at 28°C for two days. The streptomycin was included to kill the two E. coli strains. Single colonies were picked and purified by two more streakings on the above medium.

### b. Transformation of Tomato Cotyledon Tissue with pCGN7001 and pCGN2125

Substantially sterile tomato cotyledon tissue was obtained from seedlings which had been grown at 24°C, with a 16 hr/8 hr day/night cycle in 100 x 25mm Petri dishes containing Murashige-Skoog salt medium and 0.8% agar (pH 6.0). Any tomato species may be used, however, here the inbred breeding line was UC82B, available from the Department of Vegetable Crops, University of California, Davis, CA 95616. The cotyledons were cut into three sections and the middle section placed onto feeder plates for a 24-hour preincubation. The feeder plates were prepared by pipetting 0.5ml of a tobacco suspension culture (∼10⁶ cells/ml) onto 0.8% agar medium, containing Murashige minimal organic medium (K.C. Biologials), 2,4-D (0.2mg/L), kinetin (0.1mg/L), thiamine (0.9mg/L) and potassium acid phosphate (200mg/L, pH 5.5). The feeder plates were prepared two days prior to use. A sterile 3mm filter paper disk containing feeder medium was placed on top of the tobacco cells after the suspension cells had grown for two days.

Following the preincubation period, the middle segments of the cotyledon sections were placed into a liquid MG/L broth culture (1-5ml) of the Agrobacterium tumefaciens strain LBA4404/pCGN7001 or pCGN2125 (1x10⁷-1x10⁸ bacteria/ml). The cotyledon sections were cocultivated with the bacteria for 48 hrs. on the feeder plates and then transferred to regeneration medium containing 350mg/L cefotaxime and 100mg/L kanamycin. The regeneration medium is a K.C. Biologicals Murashige-Skoog salts medium with zeatin (2mg/L), myo-insitol (100mg/L), sucrose (20g/L), Nitsch vitamins and containing 0.8% agar (pH 6.0). In 2 to 3 weeks, shoots developed. When the shoots were approximately 1.25cm, they were excised and transferred to a Murashige and Skoog medium containing cefotaxime (350mg/L) and kanamycin (50mg/L for rooting. Roots developed within 10-12 days.

### 7. Analysis of GUS Expression in Transformed Tomato Seedlings

Young leaf tissue was excised from tomato plantlets that were growing from the cut edges of cotyledons that had been co-cultivated with Agrobacterium tumefaciens containing pCGN2125 or pCGN7001 constructs 6-8 weeks previously. The tissue was grown on medium containing 100mg/ml kanamycin; 350mg/ml cefotaxime (Cal Biochem-Hoechst). The remainder of the medium was 22 medium (Murashige and Skoog salts 2% sucrose, 100mg/l myo-inositol, Nitsch vitamins, 2mg/l zeatin and 0.8% Bactoagar). One plantlet from cocultivation with pCGN7001 were chosen for further analysis.

The fluorimetric GUS assay was performed essentially as described (Jefferson et al., EMBO J. (1987) 6:3901-3907). Specifically, young tomato leaf tissue (0.03-0.20 g) was ground in 500µl GUS extraction buffer (50mM sodium phosphate, pH 7.0, 10mM BME, 10mM EDTA, 0.1% Triton X-100). The extract was centrifuged in a microfuge for 10 min. and the supernatant removed. Various dilutions of the supernatant in extraction buffer were made. Typically 1:10 dilutions in 500µl total volume were analyzed. Five µl of MUG (methylumbelliferone), 100mM in dimethylformamide, were added and the reaction mixtures incubated at 37°C for 2 hours. The reaction was terminated by the addition of 900µl of 0.2 M Na₂CO₃. Fluorescence was measured with excitation at 365 nm, emission at 455 nm on a Farrand system 3 fluorimeter with slit widths set at 10 nm.

### B. Results

### 1. Characterization of a EF-1α Genomic Clone

The EF-1α gene contains one intron of 320 bp. This gene encodes a polypeptide which is 97% homologous to the polypeptide encoded by LeEF-1. 61-1 is therefore one of the members of the EF-1α multigene family, but it is not the gene that gave rise to the LeEF-1 cDNA clone. The sequence of the EF-1α genomic clone, 61-1, is shown in Figure 3.

To determine what percentage of the total EF-1 mRNA arises from the 61-1 gene and what percentage arises from the gene that gave rise to LeEF-1 (pCGN666), specific oligomers were synthesized. Oligomer EF p27 (5'-AGCTTGTAGATCAAGTCACCAGTGGTAGTC-3') corresponds to bases 123 to 153 of LeEF-1 and since it is in a conserved region of the coding sequence will bind to all EF-1α mRNAs. Oligomer EF p28 (5'-ACTGATATCTCTGGTAACTCCAGAGTTGAA-3') corresponds to bases 2542 to 2572 of 61-1 in the 3'-untranslated region. This sequence is unique to 61-1. Oligomer EF p29 (5'-ATAGCATTCGAAACACCAGCATCACACTGC-3') corresponds to bases 1413 to 1443 of LEF-1 i the 3'-untranslated region. This sequence is unique to LeEF-1.

Using these three oligomer probes, Northern analysis was run on poly(A⁺) RNA from young axial shoots of tomato. The results demonstrated that the gene that gave rise to LeEF-1 (pCGN666) contributes a major portion of the EF-1α message. Comparing signals obtained with oligomers EFp27 and EFp29, it is possible to estimate that the gene that gave rise to LeEF-1 contributes at least 30% and perhaps as much as 50-60% of the total EF-1α mRNA. On the other hand, a comparison of the signals obtained from EFp27 and EFp28, indicates that 61-1 contributes on the order of only 1% of the total EF-1α mRNA.

### 2. Analysis of Tomato Plants Transformed with pCGN2125

The results of a typical assay are shown in Table 1 below.

**Table 1.**

| FLUOROMETRIC GUS ASSAY | | | |
|---|---|---|---|
| Plant Sample | Weight of Sample | Fluorescence Intensity (F) of 1/10 Dilution | Fluorescence Intensity (F) normalized to 0.1g Sample |
| 2125-X | 0.04g | 163 | 408 |
| 7001-A | 0.14g | 311 | 222 |

The GUS levels produced by the 2125 (EF-1α GUS) plant are similar to those produced by a 7001 (mas-gus) plant. The level of EF-1α mRNA produced by the native 61-1 gene is approximately 1% of the total EF-1α RNA. Since the total EF-1α mRNA level is approximately 1% of the poly(A⁺) RNA, then the mRNA produced by the 61-1 gene represents about 0.01% of the poly(A⁺) RNA. The message produced from the mannopine synthase promoter is low level and may be equal to or less than that produced from the octopine synthase (ocs) promoter (Murai and Kemp, Nucl, Acids Res. (1982) 10:1679-1689). The level of mRNA produced from the ocs promoter averages around 0.005% and ranges from 0.002% to 0.01% of total mRNA (Comai, et al., Nature (1985) 317:741-744). Thus the mannopine promoter and the 61-1 EF-1α gene produce similar levels of mRNA. Thus the similar levels of GUS activity produced from the 2125 and 7001 plants are as would be predicted and indicate that the EF-1α-GUS fusion in 2125 is functioning as expected.

### Example III

### EF-1α Genomic Clone A, The Genomic Equivalent of pCGN666

### A. Materials and Methods

### 1. Construction of pCGN2127

The approximately 250 bp XbaI (1464) to EcoRI fragment of pCGN666 was cloned into XbaI-EcoRI digested pUC19. This resulted in pCGN2127.

### 2. Probe Description, Isolation and Labelling

Probe A: The 1.7 kb EcoRI fragment of pCGN666 containing LeEF-1 was isolated and nick-translated as previously described (Shewmaker, et al., supra).

Probe B: The approximately 250 bp XbaI-EcoRI fragment of pCGN2127 was isolated and nick-translated as previously described (Shewmaker, et al., supra). This fragment contains only sequences in the 3'untranslated portion of LeEF-1.

Probe C: The 30 base oligonucleotide EFp29 (see supra) which hybridizes to nucleotides 1413 to 1443 of LeEF-1. This oligonucleotide hybridizes to sequences in the 3' untranslated region of LeEF-1 that are different from those to which probe B hybridizes.

### 3. Screening a Tomato Genomic Library for the Genomic Equivalent to pCGN666

A tomato (Lycopersicon escultentum cv VFNT-Cherry) partial Sau3A library in Ch35 (Loenen, Gene (1983) 26:171-179) was provided by Dr. Robert Fischer of University of California, Berkeley. The basic screening procedures were as described above in Example II. When the nick-translated probes A and B were used, hybridization and washing were as described for genomic screening. When the oligonucleotide probe C was used, hybridization and washing were as described for Northern analysis using oligonucleotide probes.

Approximately 800,000 plaques of the above library were screened with probe A. Subsequent rounds of plaque purification of positive signals were done with duplicate filters, one hybridized with probe A and the other with probe B. Only plaques that hybridized to both probes were chosen for final plaque purification. Purified plaques that hybridized to both probes A and B were also screened with probe C. A clone denoted "A" that hybridized to all 3 probes was chosen for further analysis. The EF-1α gene within A was designated LeEF-A.

### 4. Mapping, Subcloning and Sequencing

DNA from phage "A" was isolated as described (Grossgerger, Nuc. Acids. Res. (1987) 15:6737). The 11 kb insert of tomato genomic DNA within "A" and the location of LeEF-A within this insert were mapped using standard techniques. (Maniatis, Fritsch and Sambrock, Molecular Cloning, A Laboratory Manual (1982) Cold Spring Harbor, New York) Several subclones of the insert in "A" were generated using standard cloning techniques. These subclones were used for DNA sequencing and included.

pCGN674 and pCGN675: The approximately 9 kb KpnI fragment cloned in both orientations in pUC19. This fragment contains 5' upstream regions as well as coding region from the amino terminal portion of the gene.

pCGN2134: The approximately 2.5 b EcoRI fragment cloned into pUC19. This fragment contains the coding region and region 3' of the LeEF-A gene.

Using pCGN674, pCGN674, pCGN2134 and standard sequencing techniques similar to those described supra, the sequence of (1) the coding region, (2) approximately 2.5 kb upstream of the initiation codon, and (3) approximately 0.7 kb 3' of the stop codon were obtained.

### 5. Construction of pCGN1558

pCGN1558 is a binary plant transformation vector containing the left and right T-DNA borders of Aarobacterium tumefaciens octopine Ti-plasmid pTiA6 (Currier and Nester J. Bact. (1976) 126:157-165), the gentamycin resistance gene of pPH1JI (Hirsch and Beringer, Plasmid (1984) 12:139-141), an Agrobacterium rhizogenes Ri plasmid origin of replication from pLJbB11 (Jouanin et al., Mol. Gen. Genet. (1985) 201:370-374), a 35S promoter-Kan^{R}-tml³ region capable of conferring kanamycin resistance to transformed plants, a ColE1 origin of replication from pBR322 (Bolivar et al., Gene (1977) 2:95-113), and a lacZ' screenable marker gene from pUC18 (Yanish-Perron, et al., *Gene* (1985) 33:103-119).

### Construction of pCGN1546

The 35S promoter-tml3' expression cassette, pCGN986 (contains a cauliflower mosaic virus 35S (CaMV35) promoter and a T-DNA tml 3'-region with multiple restriction sites between them. pCGN986 was derived from another cassette, pCGN206, containing a CaMV35S promoter and a different 3' region, the CaMV region VI 3'-end. The CaMV 35S promoter was cloned as an AluI fragment (bp 7144-7734) (Gardner et. al., Nucl. Acids Res. (1981) 9:2871-2888) into the HincII site of M13mp7 (Messing, et. al., Nucl. Acids Res. (1981) 9:309-321) to create C614. An EcoRI digest of C614 produced the EcoRI fragment from C614 containing the 35S promoter which was cloned into the EcoRI site of pUC8 (Viera and Messing, Gene (1982) 19:259) to produce pCGN147.

pCGN148a containing a promoter region, selectable marker (KAN with 2 ATG's) and 3' region, was prepared by digesting pCGN528 with BglII and inserting the BamHI-BglII promoter fragment from pCGN147. This fragment was cloned into the BglII site of pCGN528 so that the BglII site was proximal to the kanamycin gene of pCGN528.

The shuttle vector used for this construct pCGN528, was made as follows: pCGN525 was made by digesting a plasmid containing Tn5 which harbors a kanamycin gene (Jorgenson et. al., Mol. Gen. Genet. (1979) 177:65) with HindIII-BamHI and inserting the HindIII-BamHI fragment containing the kanamycin gene into the HindIII-BamHI sites in the tetracycline gene of pACYC184 (Chang and Cohen, J. Bacteriol. (1978) 134:1141-1156). pCGN526 was made by inserting the BamHI fragment 19 of pTiA6 (Thomashow et. al., Cell (1980) 19:729-739), modified with XhoI linkers inserted into the SmaI site, into the BamHI site of pCGN525. pCGN528 was obtained by deleting the small XhoI fragment from pCGN526 by digesting with XhoI and religating.

pCGN149a was made by cloning the BamHI-kanamycin gene fragment from pMB9KanXXI into the BamHI site of pCGN148a. pMB9KanXXI is a pUC4K variant (Vieira and Messing, Gene (1982) 19:259-268) which has the XhoI site missing, but contains a functional kanamycin gene from Tn903 to allow for efficient selection in Agrobacterium.

pCGN149a was digested with HindIII and BamHI and ligated to pUC8 digested with HindIII and BamHI to produce pCGN169. This removed the Tn903 kanamycin marker. pCGN565 and pCGN169 were both digested with HindIII and PstI and ligated to form pCGN203, a plasmid containing the CaMV 35S promoter and part of the 5'-end of the TN5 kanamycin gene (up to the PstI site, Jorgenson et. al., (1979), supra). A 3'-regulatory region was added to pCGN203 from pCGN204 (an EcoRI fragment of CaMV (bp 408-6105) containing the region VI 3' cloned into pUC18 (Yanisch-Perron, et al., Gene (1985) 33:103-119) by digestion with HindIII and PstI and ligation. The resulting cassette, pCGN206, was the basis for the construction of pCGN986.

The pTiA6 T-DNA tml 3'-sequences were subcloned from the Bam19 T-DNA fragment (Thomashow et al., (1980) supra) as a BamHI-EcoRI fragment (nucleotides 9062 to 12,823, numbering as in Barker et al., Plant Mol. Biol. (1982) 2:335-350) and combined with the pACYC184 (Chang and Cohen (1978), supra) origin of replication as an EcoRI-HindIII fragment and a gentamycin resistance marker (from plasmid pLB41), obtained from D. Figurski) as a BamHI-HindIII fragment to produce pCGN417.

The unique SmaI site of pCGN417 (nucleotide 11,207 of the Bam19 fragment) was changed to a SacI site using linkers and the BamHI-SacI fragment was subcloned into pCGN565 to give pCGN971. The BamHI site of pCGN971 was changed to an EcoRI site using linkers. The resulting EcoRI-SacI fragment containing the tml 3' regulatory sequences was joined to pCGN206 by digestion with EcoRI and SacI to give pCGN975. The small part of the Tn5 kanamycin resistance gene was deleted from the 3'-end of the CaMV 35S promoter by digestion with SalI and BglII, blunting the ends and ligation with SalI linkers. The final expression cassette pCGN986 contains the CaMV 35S promoter followed by two SalI sites, an XbaI site, BamHI, SmaI, KpnI and the tml 3' region (nucleotides 11207-9023 of the T-DNA).

The 35S promoter-tml 3' expression cassette, pCGN986 was digested with HindIII. The ends were filled-in with Kleenow polymerase and XhoI linkers added. The resulting plasmid was called pCGN986X. The BamHI-SacI fragment of pBRX25 containing the nitrilase gene was inserted into BamHI-SacI digested pCGN986X yielding pBRX66.

Construction of pBRX25 is described in European application EPA 0 229 042 filed January 7, 1987, which application is incorporated herein by reference. Briefly, the method was as follows: The nucleotide sequence of a 1212-bp PstI-HincII DNA segment encoding the bromoxynil-specific nitrilase contains 65-bp of 5' untranslated nucleotides. To facilitate removal of a portion of these excess nucleotides, plasmid pBRX9 was digested with PstI, and treated with nuclease Bal31. BamHI linkers were added to the resulting ends. BamHI-HincII fragments containing a functional bxn gene were cloned into the BamHI-SmaI sites of pCGN565. The resulting plasmid, pBRX25, contains only 11 bp of 5' untranslated bacterial sequence.

pBRX66 was digested with PstI and EcoRI, blunt ends generated by treatment with Kleenow polymerase, and XhoI linkers added. The resulting plasmid pBRX68 now has a tml 3' region that is approximately 1.1kb. pBRX68 was digested with SalI and SacI, blunt ends generated by treatment with Kleenow polymerase and EcoRI linkers added. The resulting plasmid, pCGN986XE is a 35S promoter - tml 3' expression cassette lacking the nitrilase gene.

The Tn5 kanamycin resistance gene was then inserted into pCGN986XE. The 1.0kb EcoRI fragment of pCGN1536 (see below) was ligated into pCGN986XE digested with EcoRI. A clone with the Tn5 kanamycin resistance gene in the correct orientation for transcription and translation was chosen and called pCGN1537b. The 35S promoter Kan^{R}-tml 3' region was then transferred to a chloramphenical resistant plasmid backbone. pCGN786 (a pUC-CAM based vector with the synthetic oligonucleotide 5' GGAATTCGTCGACAGATCTCTGCAGCTCGAGGGATCCAAGCTT 3' containing the cloning sites EcoRI, SalI, BglII, PstI, XhoI, BamHI, and HindIII inserted into pCGN566) was digested with XhoI and the XhoI fragment of pCGN1537b containing the 35S promoter - Kan^{R}-tml 3' region was ligated in. The resulting clone was termed pCGN1546.

### pCGN1536 Construction.

The 5.4 kb EcoRI fragment is removed from pVK232 (Knauf and Nester, Plasmid (1982)8:45), by EcoRI digestion and cloned into EcoRI digested pACYC184 (Chang and Cohen, J. Bacteriol. (1978) 134:1141-1156) to create pCGN14. The 1434 bp ClaI-SphI fragment of pCGN14, containing the mas 5' region (bp20128-21562 according to numbering of (Barker et al., Plant Mo. Biol. (1983) 2:335-350) is cloned into AccI-SphI digested pUC19 (Yanisch-Perron et al., Gene (1985) 33:103-119) to generate pCGN40. A 746 bp EcoRV-NaeI fragment of the mas 5' region is replaced by an XhoI site by digesting pCGN40 with EcoRV and NaeI followed by ligation in the presence of a synthetic XhoI linker DNA to create pCGN1036. The 765 bp SstI-HindIII fragment (bp 18474-19239) of pCGN14, containing the mas 3' region, is cloned into SstI-HindIII digested pUC18 (Yanish-Perron et al., Gene (1985) 33:103-119) to yield pCGN43. The HindIII site of pCGN43 is replaced with an EcoRI site by digestion with HindIII, blunt ending with Klenow enzyme, and ligation of synthetic EcoRI linker DNA to create pCGN1034. The 767 bp EcoRI fragment of pCGN1034 is cloned into EcoRI-digested pCGN1036 in the orientation that places bp 19239 of the mas 3' region proximal to the mas 5' region to create pCGN1040. pCGN1040 is subjected to partial digestion with SstI, treated with T4 DNA polymerase to create blunt ends, and ligated in the presence of synthetic XhoI linker DNA; a clone is selected in which only the SstI site at the junction of bp 18474 and vector DNA (constructed in pCGN43 and carried into pCGN1040) is replaced by an XhoI site to generate pCGN1047.

pCGN565 is digested with EcoRI and HindIII, treated with Klenow enzyme to create blunt ends, and ligated in the presence of synthetic XhoI linker DNA to create pCGN1003; this recreates the EcoRI site adjacent to the XhoI linker. pCGN1003 is digested with EcoRI, treated with Klenow enzyme to create blunt ends, and ligated in the presence of synthetic PstI linker DNA to create pCGN1007. The 1.5kb XhoI fragment of pCGN1047, containing the mas 5' region and the mas 3' region with a multiple cloning site between, is cloned into XhoI digested pCGN1007 to construct pCGN1052. A portion of the multiple cloning site of pCGN1052 is deleted by digestion with XbaI and SstI, treated with Klenow enzyme to make blunt ends, and ligated to generate pCGN1052ΔXS.

The 1 kb EcoRI-SmaI fragment of pCGN550 (See pCGN783, Example II), containing the 1 ATG-kanamycin resistance gene, is cloned into EcoRI-SmaI digested Bluescript M13-KS (Strategene, Inc., CA) to create pBSKm; this plasmid contained an M13 region allowing generation of single stranded DNA. Single stranded DNA is generated according to the supplier's recommendations, and in vitro mutagenesis was performed (Adelman et al., DNA (1983) 2:183-193) using a synthetic oligonucleotide with the sequence 5'GAACTCCAGGACGAGGC3' to alter a PstI site with the kanamycin resistance gene and make it undigestable, creating pCGN1534. pCGN1534 is digested with SmaI and ligated in the presence of synthetic EcoRI linker DNA to generate pCGN1535.

The 1 kb EcoRI fragment of pCGN1535 is cloned into EcoRI digested pCGN1052ΔXS to create the mas5'-kan mas3' plant selectable marker cassette pCGN1536.

### Construction of pCGN1541b

pCGN65α2XX (see below) was digested with BglII and EcoRV, treated with Kleenow polymerase, and BglII linkers added. The resulting plasmid, pCGN65α2XX', now lacks an approximately 20 bp piece of DNA that was present in pCGNα2XX.

pBR322 (Bolivar et al., Gene (1977) 2:95-113) was digested with EcoRI and PvuII, treated with Kleenow polymerase to generate blunt ends, and BglII linkers added. An ampicillin resistant, tetracycline sensitive clone, pCGN1538 was selected. This clone now lacks the approximately 2.2 kb EcoRI-PvuII fragment containing the tetracycline resistance gene. The PvuII site has been lost but the EcoRI site was regenerated upon addition of BglII linkers.

pCGN65α2XX' was digested with BglII and ligated to BglII digested pCGN1538 to create pCGN1541a which contained both plasmid backbones. pCGN1541a was digested with XhoI and religated. An ampicillin resistant, chloramphenical sensitive clone was chosen which lacked the pACYC-184 derived backbone, creating pCGN1541b.

### Construction of pCGN65α2XX

pCGN451 consists of octopine synthase 5' non-coding region sequences 13,643 to 15,208 fused via an EcoRI linker to the 3' non-coding region sequences 11,207 to 12,823 of pTiA6 according to Barker et al., Plant Mol. Biol. (1983) 2:325. pCGN451 was digested with HpaI and ligated in the presence of synthetic SphI linker DNA to generate pCGN55. The XhoI-SphI fragment of pCGN55 (bp 13800-15208, including the right border, of Agrobacterium tumefaciens T-DNA; (Barker et al., Gene (1977) 2:95-113) was cloned into SalI-SphI digested of pUC19 (Yanisch-Perron et al., Gene (1985) 33:103-119) to create pCGN60. The 1.4 kb HindIII-BamHI fragment of pCGN60 was cloned into HindIII-BamHI digested with pSP64 (Promega, Inc.) to generate pCGN1039. pCGN1039 was digested with SmaI and NruI (deleting bp14273-15208; (Barker et al., Gene (1977) 2:95-113) and ligated in the presence of synthetic BglII linker DNA creating pCGN1039ΔNS. The 0.47 kb EcoRI-HindIII fragment of pCGN1039ΔNS was cloned in to EcoRI-HindIII digested pCGN565 to create pCGN565RB. The HindIII site of pCGN565RB was replaced with an XhoI site by HindIII digestion, treatment with Klenow enzyme, and ligation in the presence of synthetic XhoI linker DNA to create pCGN565RB-H+X.

pUC18 was digested with HaeII to release the *lac*Z' fragment, treated with Klenow enzyme to create blunt ends, and the *lac*Z'-containing fragment ligated into pCGN565RB-H+X, which had been digested with AccI and SphI and treated with Klenow enzyme, resulting in pCGN565RB α2X. In pCGN565RBα2X the orientation of *lac*Z' is such that the *lac* promoter is proximal to the right borderThe clone is positive for *lac*Z' expression when plated on an appropriate host and contains bp 13990-14273 of the right border fragment (Barker et al. (1983) supra), having deleted the AccI-SphI fragment (bp 13800-13990). The 728 bp BglII-XhoI fragment of pCGN565RBα2X containing the T-DNA right border piece and the *lac*Z*'* gene, was cloned into BglII-XhoI digested pCGN65ΔKX-S+X, replacing the BglII-XhoI right border fragment of pCGN65ΔKX-S+X, to create pCGN65α2X.

### Construction of pCGN65ΔKX-S+X

pCGN501 was constructed by cloning a 1.85 kb EcoRI-XhoI fragment of pTiA6 (Currier and Nester, J. Bact. (1976) 126:157-165) containing bases 13362-15208 (Barker et al., Plant Mo. Biol. (1983) 2:335-350) of the T-DNA (right border), into EcoRI-SalI digested M13mp9 (Vieira and Messing, Gene (1982) 19:259-268). pCGN502 was constructed by cloning a 1.6 kb HindIII-SmaI fragment of pTiA6, containing bases 602-2212 of the T-DNA (left Border), into HindIII-SmaI digested M13mp9. pCGN501 and pCGN502 were both digested with EcoRI and HindIII and both T-DNA-containing fragments cloned together into HindIII digested pUC9 (Vieira and Messing, Gene (1982) 19:259-268) to yield pCGN503, containing both T-DNA border fragments. pCGN503 was digested with HindIII and EcoRI and the two resulting HindIII-EcoRI fragments (containing the T-DNA borders) were cloned into EcoRI digested pHC79 (Hohn and Collins, Gene (1980) 11:291-298) to generate pCGN518. The KpnI-EcoRI fragment from pCGN518, containing the left T-DNA border, is cloned into KpnI-EcoRI digested pCGN565 to generate pCGN580. The BamHI-BglII fragment of pCGN580 is cloned into the BamHI site of pACYC184 (Change and Cohen, J. Bacteriol. (1978) 134:1141-1156) to create pCGN51. The 1.4 kb BamHI-SphI fragment of pCGN60 (see pCGN65α2X section above) containing the T-DNa right border fragment, was cloned into BamHI-SphI digested pCGN51 to create pCGN65.

pCGN65 was digested with KpnI and XbaI, treated with Klenow enzyme to create blunt ends, and ligated in the presence of synthetic BglII linker DNA to create pCGN65ΔKX. pCGN65ΔKX was digested with SalI, treated with Klenow enzyme to create blunt ends, and ligated in the presence of synthetic XhoI linker DNA to create pCGN65ΔKX-S+X.

### Final Construction of pCGN1558

The XhoI fragment of pCGN1546 containing the 35S promoter - Kan^{R}-tml 3' region was cloned into XhoI digested 1541b to create pCGN1554. The XhoI fragment from pCGN1546 is oriented within 1541b such that the order of components is <left border - tml 3' - Kan^{R} - 35S promoter - lacZ'>

### <right border.

The BglII fragment of pCGN1554 containing left border-tml3'-Kan^{R}-35S promoter-lacZ right border was cloned into BamHI restricted pCGN1532 (see below) resulting in the binary vector, pCGN1558. In pCGN1558, the orientation of the insert is such that the T-DNA left border is adjacent to the RI plasmid origin of replication. This binary vector has several advantages including a minimal amount of DNA between the T-DNA borders, high stability in Agrobacterium hosts, high copy number in E. coli hosts and blue/white screen with multiple restriction enzyme sites for ease of cloning target DNA.

### pCGN1532 construction.

The 3.5kb EcoRI-PstI fragment containing the gentamycin resistance gene is removed from pPhlJI (Hirsch and Beringer, Plasmid (1984) 12:139-141) by EcoRI-PstI digestion and cloned into EcoRI-PstI digested pUC9 (Vieira and Messing, Gene (1982) 19:259-268) to generate pCGN549. HindIII-PstI digestion of pCGN549 yields a 3.1 kb fragment bearing the gentamycin resistance gene, which is made blunt ended by the Klenow fragment of DNA polymerase I and cloned into PvuII digested pBR322 (Bolivar et al., Gene (1977) 2:95-113) to create pBR322Gm. pBR322Gm was digested is DraI and SphI, treated with Klenow enzyme to create blunt ends, and the 2.8 kb fragment cloned into the Ri origin containing plasmid pLJbB11 (Jouanin et al., Mol. Gen. Genet. (1985) 201:370-374) which has been digested with ApaI and made blunt ended with Klenow enzyme, creating pLHbB11Gm. The extra ColE1 origin and the kanamycin resistance gene are deleted from pLJvB11GM by digestion with BamHI followed by self closure to create pGmB11. The HindII site of pGmB11 is deleted by HindII digestion followed by treatment with Klenow enzyme and self closure, creating pGmB11-H. The PstI site of pGmB11-H is deleted by PstI digestion followed by treatment with Klenow enzyme and self closure, creating pCGN1532.

### 6. Construction of EF-1α (Le-EFA)-GUS Construct and Plant Transformation

### a. Construction of pCGN2147 - EF-1α 3' subclone for use in EF-1α cassette

pCGN2141 was constructed by cloning the approximately 1.8kb KpnI-PstI fragment of pCGN2134 into pUC19 cut with KpnI x PstI. This KpnI (Figure 4, nucleotide #2771) - PstI fragment contains coding and 3' untranscribed regions. The approximately 1.0kb BglII-PstI fragment of 2141 was cloned into Bluescript KSII (Stratagene) that has been digested with BamHI x PstI yielding pCGN2145. This BglII (Figure 4, nucleotide #3619) - Pst fragment contains C-terminal coding regions and 3' untranscribed regions. The 1.0kb SstI-EcoRI fragment of 2145 was cloned into SstI-EcoRI digested pCGN565 yielding pCGN2147.

### b. Construction of 2140-EF-1α 5' Promoter Subclone For Use In EF-1α Cassette

In order to obtain desirable restriction sites for cloning in genes at the 3' end of a promoter fragment, a small fragment of the 5' region of EF-1α was amplified using polymerase chain reaction (PCR) (Perkin-Elmer, Cetus) thermal cycler, as per the manufacturer's instructions (including manufacturer's reagents) and oligonucleotides that add desired linkers. This small fragment was then rejoined with other 5' sequences to make a suitable promoter fragment. Specifically the oligos were:

EFp54
HindIII
|---------|
5'   CAG - CAG - AAG - CTT - GCT - GTA - GTT - GTT-
2135

TAC - CAG 3'
2158

EFp56

Sst   Sma
|--------|  |--------|
5'   ACG - ACG - GAG - CTC - TCG - CCC - GGG

Xho
|-------|
- CAT - CTC - GAG - ACA - CTA - AGA - AAC
2450

TGC - ATT - TAC - 3'
2427

Specifically the reaction contained 53.5ul H₂O, 10ul 10xRxn buffer, 16ul dNTP's [1.25mM dCTP, dATP, dGTP & TTP], 5ul EF-p54 (20mM), 5ul EF-p56 (20mM), 20ul pCGN675 (1ng/ul), 0.5ul TAQ polymerase. The resulting ≈ 300bp fragment was restricted with HindIII and SstI and cloned into pUC12 Cm^{R} (Buckley, Ph.D. thesis) that had been cut with HindIII and SstI. This resulted in pCGN2142. The sequence of the fragment in 2142 generated by PCR was determined and found to be identical to the same region in pCGN675 (See Example 3) (Figure 4, nucleotide #2135--> 2450).

pCGN2137 was obtained by cutting pUC19 with HindIII, filling in with Kleenow polymerase and selecting for a clone that had lost the HindIII site.

The approximately 6.5kb BamHI-KpnI (2771) fragment of pCGN675 was cloned into BamHI x KpnI cut pCGN2137 yielding pCGN2139. The BamHI-KpnI fragment in pCGN2139 contains 5' upstream regions and approximately 300 base pairs of N-terminal coding region. The approximately 9.5kb HindIII-SstI fragment of 2139 was ligated to the approximately 300bp HindIII-SstI fragment of pCGN2142 yielding pCGN2140. The approximately 6kb EF-1α 5' fragment in 2140 contains 5' upstream and 5' untranslated regions but no coding sequences.

### c. Construction of pCGN2148-EF-1α Cassette

The approximately 6kb SphI-SstI fragment of 2140 containing the EF-1α promoter was cloned into SphI-Sst cut pCGN2147 yielding pCGN2148. This clone thus contains the approximately 6kb 5' promoter fragment, the approximately 1kb 3' EF-1α fragment and restriction sites in between into which genes can be cloned.

### d. Construction of pCGN2154-EF-1α-GUS Chimera

pCGN1804 (see Example II) contains the E. coli β-glucuronidase (GUS) gene. pCGN1804 was cut with EcoRI and the ends filled in with polymerase I (Focus (1984) 6:1 pg. 6). XhoI linkers were added and a clone with an XhoI site was selected and designated pCGN1805. The 1.8kb SalI x XhoI fragment of pCGN1805 containing the GUS gene was cloned into XhoI digested pCGN2148. A clone with the GUS gene in the correct orientation for transcription and translation was selected and given the number pCGN2154.

### e. Insertion of EF-1α GUS Chimera (pCGN2154) Into A Binary Vector (pCGN1558)

The approximately 9kb PstI fragment of pCGN2154 containing EF-1α 5'-GUS-EF-1α3' was cloned into PstI cut pCGN1558 (see above). In pCGN2158 the 5'EF-1α-GUS EF-1α3' was inserted into pCGN1558 such that 5'EF-1α-GUS-EF-1α3' is transcribing in the same direction as the 35S-Kan-tml gene. In pCGN2157 the 5'EF-1α-GUS-EF-1α3' was inserted into pCGN1558 such that 5'EF-1α-GUS-EF-1α3' is transcribing in the opposite direction as the 35S-Kan-tml gene.

### f. Preparation of Transgenic Plants

Agrobacterium tumefaciens strain 2760 (also known as LBA4404, Hoekerana et al., Nature (1983) 303:179-180) was transformed with pCGN2157 or pCGN2158 using the method of Holsters, et al., Mol. Gen. Genet., (1978) 163:181-187. The transformed A. tumefaciens was then used in the co-cultivation of plants.

### Tomato

Tomato plants were transformed with A. tumefaciens LBA4404/pCGN2158 or pCGN2157, respectively, essentially as described in Example II. 6.b., with the exception that 500 mg/L of carbenicillin was used in the co-cultivation and regeneration procedure instead of the 350 mg/l cefotoxine described therein.

### Tobacco

Tobacco leaf explants, roughly 5-10mm by 5-10mm, were cut from young leaves, approximately 3-5cm long and third to sixth from the apex of *Nicotiniana tbabcum* cv *xanthi* which were grown under axenic conditions in solid medium: Murashige Minimal Organics (#1118 Gibco Laboratories, New York), 7% phytagar, 1mg/l indole-3-acetic acid, 0.15mg/l kinetin. The explants were plated on solid medium containing Murashige Minimal Organics, 6% phytagar, 40mg/l adenine sulfate, 2mg/l indole-3-acetic acid, 2mg/l kinetin. A sterile #1 Whatman filter paper (Whatman Ltd., Maidstone, England) was placed on the top of the plate medium (explants are placed on top of filter) and they were incubated for 24 hours in the dark at 24°C.

The *Agrobacterium* were grown on AB medium (AB salts K₂HPO₄ 3gm/l, NaH₂PO₄ H₂O 1.15g/l, NH₄Cl 1g/l, glucose 5g/l, FeSO₄ 0.25mg/l, MgSO₄ 0.246mg/l, 0.14mg/l, 15g/l agar, 100 ug/l gentamycin sulfate and 100 ug/l streptomycin sulfate) for 4-5 days. Single colonies were inoculated into 5mls of MG/L broth (50% Luria broth and 50% mannitol-glutamate salts medium (Garfinkel and Nester, *J.Bacteriol.* (1980)144:732-743)) and were incubated overnight in a shaker at 30°C and 180 R.P.M. before co-cultivation.

Following the preincubation period, the explants were dipped into the bacterial suspension of 3.3 x 10⁸ cells/ml for approximately 5 minutes, blotted on sterile paper towels and replated on the same plates. After 48 hours, the explants were placed on selection medium containing the same plate medium as above plus 350mg/l cefotaxime and 100mg/l kanamycin. The explants were transferred to fresh media every 2 weeks. At the 6 week transfer thereafter, shoot and green callus were trimmed from explants and placed on solid media: Murashige Minimal Organics, .5mg/l indole-3-acetic acid, 2 mg/l kinetin, 40mg/l adenine sulfate, 350mg/l cefotaxime, 100mg/l kanamycin. Shoots were harvested beginning about 4 weeks after co-cultivation and placed in 50ml culture tubes with 25ml of solid medium (7% bactagar 1mg/l indole-3-butyric acid, 350mg/l cefotaxime, 100mg/l kanamycin) and grown at 24-28°C, 12 hours light, 12 hours dark, light intensity 80-100uEm⁻²s⁻¹. Shoots root in 1-2 weeks and were then transplanted into soil and placed in growth chambers.

### B. Results

### 1. Characterization of EF-1α Genomic Clone A (LeEF-A)

This sequence, comprising 4565 nucleotides in total is presented in Figure 4. Notable features of the sequence include:
a. The EF-1α gene A has 2 introns (indicated in small letters). One occurs in the 5' untranslated region between nucleotides 43 and 44 of the cDNA (pCGN666). This intron spans nucleotides 1680-2436 of this sequence. The second intron occurs in the coding region between amino acids 154 and 155. This intron spans nucleotides 2916-2995.
b. The 5' upstream region of the LeEF-A gene is rather rich in A/T. The 2453 nucleotides upstream of the initiation ATG are 75.1% A/T. When the intron within this region (757 nucleotides) is examined, it is found to be 70.4% A/T. This contrasts to the 1347 nucleotides in the coding region (excluding intron 2) which shows 52.2% A/T.

### 2. GUS Analysis of pCGN2157/pCGN2158 Transformed Plants

Transformed tobacco plants were maintained in vitro on media containing 0.7% Phytagar (Gibco), Murashige and Skoog salts, 3% sucrose, 1 mg/l IAA, and 0.15 mg/l kinetin, pH 5.5. Transformed tomato plants were maintained in vitro on media containing 0.65% Phytagar, Murashige and Skoog salts, 1x Nitsch vitaimins (Nitsch, C. and Nitsch, J.P. (1967) Planta (Berl) 72:355-370), 0.15 mg/l kinetin, pH 6.0.

### a. Flurometric Analysis

Analysis was performed on very young tobacco shoot tips. Shoot tips, weighing approximately 50-100 mg were quick frozen in liquid nitrogen and stored at -70° C. until assayed. Samples were ground in 500 µl GUS extraction buffer as described in Example II.7. In addition, protein concentration was determined on a separate aliquot using the Bio-Rad Protein Assay Kit (Bio-Rad) with Bovine Serum Albumyn as standards. The data was then converted to pmole MU/min/mg protein.

### b. Localization of GUS Activity Within Tissue

In situ GUS analysis was performed with X-glucuronic acid (Molecular Probes, Inc., OR) as described in (Jefferson, Plant Mol. Biol. Reporter (1987) 5:387-405). For the tobacco shoot tip, free hand sections approximately 10-20 cell layers thick were made and immersed in staining solution overnight at 37°C. For tomato root tips, young roots were immersed in staining solution overnight at 37°C.

### 3. Expression Of EF-1α Gene A-GUS Chimera

To determine the pattern of expression of the EF-1α-GUS chimera, in situ hybridizations were performed using X-glucuronic acid. The action of B-glucuronidase on X-glucuronic acid gives rise to a blue colored compound.

A young root tip from a pCGN2157 tomato plantlet and a pCGN7001 (mas-GUS chimera - See Example II) plantlet were examined and compared. In the pCGN7001 root tip, development of color is seen throughout the root. In the pCGN2157 root, the main color development is seen in the very tip of the root. When endogenous EF-1α expression was analyzed in tomato root tips by 35S - RNA in situ hybridization (See, Example 1 and Pokalsy et al., (1989) Nuc. Acids Res., 17:4661-4673) the majority of the hybridization was seen in the very root tip (1st 1-2 mm). The expression seen with pCGN2157 is similar and correlates with the rapid growth that is taking place in the root tip.

A shot tip from a pCGN2157 tobacco plant was sectioned and treated with X-glucuronic acid. The development of color is seen in the very apex of the shoot, the procambium, and the axillary buds. These regions are characterized by rapidly dividing cells. This indicates that the pattern for the EF-1α-GUS chimera correlates with area of rapid cell division.

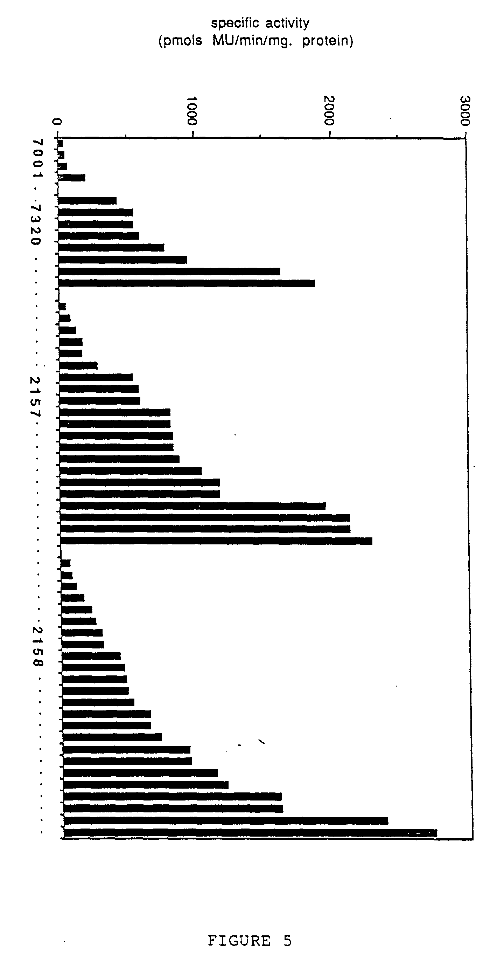

Levels of expression were analyzed by looking at GUS levels in young shoot tips. Twenty pCGN2157 and twenty-four pCGN2158 tobacco plants were analyzed (See, Figure 5). Expression levels of tested events were compared to different pCGN7001 tobacco plant events (mas-GUS chimera, See, Example II) and against pCGN7320 repeated testing of a clonal transformation event, clone pCGN7320 (double 35S-GUS chimera). The "2157" and "2158" plants showed GUS levels similar to or higher than the "d35S" plant and significantly higher than the "mas plants." Mas is known to be a relatively low level expresser (See, Example II) while d35S promoters give relatively high level expression (Kay et al. (1987) Science, 236:1299-1302). The level of expression from observed pCGN2157 and pCGN2158 correlate well with the fact that EF-1α genomic clone A is high expressing EF-1α gene.

It is evident from the above results, that plant cells and plants can be produced which have improved properties or may produce a desired product. In accordance with the subject invention, rapidly dividing cells can provide for high levels of a desired product, which may impart resistance of the plant to a wide variety of environmental hazards, such as herbicides, pests, heat, drought, disease, other forms of stress, and the like. In addition, the rate of growth of the plant may be modulated, so that higher or lower growth rates can be achieved, depending upon the particular situation. In this manner, crops may be grown more efficiently, in the substantial absence of weeds, may be less subject to attack by pathogens, so as to provide for enhanced productivity and greater efficiency in utilization of land and chemical resources, and the like. The subject constructs provide for enhanced expression of the particular gene in rapidly dividing cells, so as to reduce the level of expression in those parts of the plant where the expression product is not efficiently utilized. This provides for more efficient utilization of plant energy and resources, so that the energy and resources may be directed to a product of interest.

All publications and patent applications mentioned in this specification are indicative of the level of skill of those skilled in the art to which this invention pertains.

Although the foregoing invention has been described in some detail by way of illustration and example for purposes of clarity of understanding, it will be obvious that certain changes and modifications may be practiced within the scope of the appended claim.

## Claims

1. A DNA sequence comprising the 5' non-coding region from a plant elongation factor-1α gene according to Figure 3 or Figure 4 wherein the 5' non-coding region comprises the transcriptional and translational regulatory region and the DNA sequence is free of an intact elongation factor-1α gene coding region.

2. A DNA sequence according to claim 1 comprising the transcriptional and translational regulatory region of the sequence shown in Figure 4.

3. The DNA sequence according to claim 1, where said 5' non-coding region is less than about 10kb.

4. The DNA sequence according to claim 1, wherein said sequence has fewer than 20 codons of said coding region.

5. The DNA sequence according to claim 1, wherein said plant is tomato.

6. A DNA construct comprising in the direction of transcription,
a DNA sequence comprising a 5' non-coding region according to any one of claims 1 to 5
a DNA sequence of interest other than a sense sequence of a plant elongation factor 1-α gene; and
a 3' non-coding region comprising transcriptional and translational termination regions functional in a plant cell,
wherein expression of said DNA sequence of interest is regulated by said non-coding regions.

7. The DNA construct according to claim 6, wherein said 3' non-coding region is obtainable from a plant elongation factor 1-α gene.

8. The DNA construct according to claim 6, wherein said DNA sequence of interest is a structural gene.

9. The DNA construct according to claim 6, wherein said DNA sequence of interest is an antisense sequence.

10. The DNA construct according to claim 9, wherein said antisense sequence encodes a plant elongation factor-α or fragment thereof of at least about 15 nucleotides.

11. The DNA construct according to claim 6, wherein said DNA sequence encodes an enzyme.

12. The DNA construct according to claim 11, wherein the enzyme is 5-enolpyruvyl-2-phosphoshikimate synthase.

13. An expression cassette for integration into a plant genome comprising a DNA construct according to any one of claims 6 to 12.

14. The expression cassette according to claim 13, further comprising a marker for selection of transformed plant cells.

15. A plant cell comprising a DNA sequence comprising
a DNA construct according to any one of claims 6 to 12.

16. A plant comprising a plant cell according to claim 15.

17. A plant according to claim 16 which is a tomato plant.

18. A plant according to claim 16 or claim 17 which is other than an individual member of a "plant variety".

## Patentansprüche

1. DNA-Sequenz, welche die 5'-Nichtkodierungsregion aus einem Pflanzenverlängerungsfaktor-1α-Gen gemäß Fig. 3 oder Fig. 4 umfasst, worin die 5'-Nichtkodierungsregion die Transkriptions- und Translationsregulationsregion umfasst und die DNA-Sequenz frei von einer intakten Verlängerungsfaktor-1α-Gen-Kodierungsregion ist.

2. DNA-Sequenz nach Anspruch 1, welche die Transkriptions- und Translationsregulationsregion mit der in Fig. 4 dargestellten Sequenz umfasst.

3. DNA-Sequenz nach Anspruch 1, worin die 5'-Nichtkodierungsregion kürzer als etwa 10 kb ist.

4. DNA-Sequenz nach Anspruch 1, worin die Sequenz weniger als 20 Codons der Kodierungsregion aufweist.

5. DNA-Sequenz nach Anspruch 1, worin die Pflanze eine Tomatenpflanze ist.

6. DNA-Konstrukt, das in Transkriptionsrichtung Folgendes umfasst:
eine DNA-Sequenz, die eine 5'-Nichtkodierungsregion nach einem der Ansprüche 1 bis 5 umfasst,
eine DNA-Sequenz von Interesse mit Ausnahme einer Sense-Sequenz eines Pflanzenverlängerungsfaktor-1α-Gens; und
eine 3'-Nichtkodierungsregion, die in einer Pflanzenzelle funktionelle Transkriptions- und Translationsterminationsregionen umfasst,
worin die Expression der DNA-Sequenz von Interesse durch die Nichtkodierungsregionen reguliert wird.

7. DNA-Konstrukt nach Anspruch 6, worin die 3'-Nichtkodierungsregion von einem Pflanzenverlängerungsfaktor-1α-Gen erhältlich ist.

8. DNA-Konstrukt nach Anspruch 6, worin die DNA-Sequenz von Interesse ein Strukturgen ist.

9. DNA-Konstrukt nach Anspruch 6, worin die DNA-Sequenz von Interesse eine Antisense-Sequenz ist.

10. DNA-Konstrukt nach Anspruch 9, worin die Antisense-Sequenz für einen Pflanzenverlängerungsfaktor-α oder ein Fragment davon mit zumindest etwa 15 Nukleotiden kodiert.

11. DNA-Konstrukt nach Anspruch 6, worin die DNA-Sequenz für ein Enzym kodiert.

12. DNA-Konstrukt nach Anspruch 11, worin das Enzym 5'-Enolpyruvyl-2-phosphoshikimate-Synthase ist.

13. Expressionkassette zur Integration in ein Pflanzengenom, die ein DNA-Konstrukt nach einem der Ansprüche 6 bis 12 umfasst.

14. Expressionskassette nach Anspruch 13, die weiters einen Marker zur Selektion transformierter Pflanzenzellen umfasst.

15. Pflanzenzelle, die eine DNA-Sequenz umfasst, die ein DNA-Konstrukt nach einem der Ansprüche 6 bis 12 umfasst.

16. Pflanze, die eine Pflanzenzelle nach Anspruch 15 umfasst.

17. Pflanze nach Anspruch 16, die eine Tomatenpflanze ist.

18. Pflanze nach Anspruch 16 oder 17, die kein einzelner Vertreter einer "Pflanzenvarietät" ist.

## Revendications

1. Séquence d'ADN comprenant la région non codante côté 3' d'un gène d'un facteur 1α d'allongement de plante selon la Figure 3 ou la Figure 4 où la région non codante côté 5' comprend la région de régulation de transcription et de traduction et la séquence d'ADN est exempte d'une région de codage du gène du facteur-1α d'allongement intacte.

2. Séquence d'ADN selon la revendication 1 comprenant la région de régulation de transcription et de traduction de la séquence montrée à la Figure 4.

3. séquence d'ADN selon la revendication 1, où ladite région non codante côté 5' a moins d'environ 10kb.

4. Séquence d'ADN selon la revendication 1, où ladite séquence a moins de 20 codons de ladite région codante.

5. Séquence d'ADN selon la revendication 1, où ladite plante est la tomate.

6. Construction d'ADN comprenant dans la direction de transcription,
une séquence d'ADN comprenant une région non codante côté 5' selon l'une quelconque des revendications 1 à 5
une séquence d'ADN d'intérêt autre qu'une séquence sens d'un gène d'un facteur 1-α d'allongement de plante; et
une région non codante côté 3' comprenant les régions de terminaison de transcription et de traduction fonctionnelles dans une cellule de plante,
où l'expression de ladite séquence d'ADN d'intérêt est régulée par lesdites régions non codantes.

7. Construction d'ADN selon la revendication 6, où ladite région non codante côté 3' peut être obtenue d'un gène d'un facteur 1-α d'allongement de plante.

8. Construction d'ADN selon la revendication 6, où ladite séquence d'ADN d'intérêt est un gène de structure.

9. Construction d'ADN selon la revendication 6, où ladite séquence d'ADN d'intérêt est une séquence antisens.

10. Construction d'ADN selon la revendication 9, où ladite séquence antisens code pour un facteur-α d'allongement de plante ou son fragment d'au moins environ 15 nucléotides.

11. Construction d'ADN selon la revendication 6, où ladite séquence d'ADN code pour une enzyme.

12. Construction d'ADN selon la revendication 11, où l'enzyme est la 5-énolpyruvyl-2-phosphoshikimate synthase.

13. Cassette d'expression pour intégration dans un génome de plante, comprenant une construction d'ADN selon l'une quelconque des revendications 6 à 12.

14. Cassette d'expression selon la revendication 13, comprenant de plus un marqueur pour la sélection des cellules transformées de plante.

15. Cellule de plante comprenant une séquence d'ADN comprenant
une construction d'ADN selon l'une quelconque des revendications 6 à 12.

16. Plante comprenant une cellule de plante selon la revendication 15.

17. Plante selon la revendication 16 qui est une plante de tomate.

18. Plante selon la revendication 16 ou la revendication 17 qui est autre qu'un membre individuel d'une "variété de plante".
